(19) Europäisches Patentamt
European Patent Office
Office européen des brevets

(11) Veröffentlichungsnummer: **0 042 593**
**B1**

(12) **EUROPÄISCHE PATENTSCHRIFT**

(45) Veröffentlichungstag der Patentschrift: **03.04.85**

(21) Anmeldenummer: **81104680.4**

(22) Anmeldetag: **19.06.81**

(51) Int. Cl.$^4$: **C 07 D 215/46,**
C 07 D 473/34,
C 07 D 213/74,
C 07 D 231/52,
C 07 D 231/54,
C 07 D 231/56,
C 07 D 239/95,
C 07 D 239/42,
C 07 D 241/44, A 61 K 31/395
// C07C93/06, C07C93/14

# (54) Aryloxypropanolamine, Verfahren zu ihrer Herstellung und diese Verbindungen enthaltende Arzneimittel.

(30) Priorität: **23.06.80 DE 3023369**

(43) Veröffentlichungstag der Anmeldung:
**30.12.81 Patentblatt 81/52**

(45) Bekanntmachung des Hinweises auf die Patenterteilung:
**03.04.85 Patentblatt 85/14**

(84) Benannte Vertragsstaaten:
**AT BE CH DE FR GB IT LI LU NL SE**

(56) Entgegenhaltungen:
**DE-A-2 814 168**
**DE-A-2 819 629**
**DE-A-2 844 497**

(73) Patentinhaber: **BOEHRINGER MANNHEIM GMBH**
**Patentabteilung, Abt. E Sandhofer Strasse 112-132 Postfach 31 01 20**
**D-6800 Mannheim 31 Waldhof (DE)**

(72) Erfinder: **Wiedemann, Fritz, Dr.**
**Weinheimer Strasse 82**
**D-6940 Weinheim-Lützelsachsen (DE)**
Erfinder: **Kampe, Wolfgang, Dr.rer.nat.**
**Zedernstrasse 49**
**D-6805 Heddesheim (DE)**
Erfinder: **Dietmann, Karl, Prof.Dr.**
**Eisenacher Weg 75**
**D-6800 Mannheim 31 (DE)**
Erfinder: **Sponer, Gisbert, Dr.**
**Lessingstrasse 13**
**D-6941 Laudenbach (DE)**

**Die Akte enthält technische Angaben, die nach dem Eingang der Anmeldung eingereicht wurden und die nicht in dieser Patentschrift enthalten sind.**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 042 593 B1

Courier Press, Leamington Spa, England.

**Beschreibung**

Die vorliegende Erfindung betrifft Aryloxypropanolamine der nachstehend genannten Formeln, Verfahren zu ihrer Herstellung sowie Arzneimittel zur Bekämpfung und Prophylaxe von Herz- und Kreislauferkrankungen, die diese Verbindungen enthalten.

Ältere Menschen leiden häufig daran, daß das Herz nicht mehr die Leistung erbringt, die nötig ist, um alle Organe des Körpers ausreichend mit Blut zu versorgen (= Altersherz, Herzinsuffizienz). Diese Minderleistung des Herzens ist dadurch gekennzeichnet, daß sich das Herz nicht mehr genügend kräftig kontrahiert und somit nicht genug Blut auswirft. Dementsprechend können Substanzen, die die Kontraktionskraft (= Inotropie) steigern, beim Altersherzen günstige Wirkungen entfalten.

Neben den bekannten Digitalisglykosiden, die wegen ihrer toxischen Wirkung schwierig anzuwenden sind, wirken Sympathomimetika am Herzen kontraktionskraftsteigernd. Jedoch ist der Nachteil dieser Stoffe im allgemeinen, daß sie gleichzeitig die Herzschlagfrequenz erhöhen, wodurch das Herz stark belastet wird (erhöhter Sauerstoffverbrauch). Dadurch sind diese Stoffe nur begrenzt einsetzbar.

Gegenüber ähnlichen Verbindungen, wie sie in den DE—A—28 19 629 und DE—A—28 44 497 beansprucht sind, kann mit den Verbindungen der vorliegenden Erfindung eine überraschende Wirkungsverbesserung erzielt werden, da diese am Herzen kontraktionssteigernd wirken, aber die Herzschlagfrequenz nur wenig beeinflussen.

Die Kontraktionskraft des Herzens kann man messen, indem man den Druck in der linken Hauptkammer (linker Ventrikel) mißt und nach der Zeit differenziert (dp/dt). Ausgewertet wird hierbei die bei jedem Herzschlag ermittelte maximale Druckanstiegsgeschwindigkeit.

Ein tierexperimentelles Modell mit einem typischen "Altersherzen" existiert nicht. Um aber die Bedingungen, die beim Menschen existieren, nachzuahmen, kann man an wachen Versuchstieren (z.B. Hunden) den Einfluß von Prüfsubstanzen auf die Kontraktilität des Herzens (dp/dt) und die Herzschlagfrequenz ($f_{cor}$) prüfen. Für einen eventuellen therapeutischen Einsatz eignen sich besonders solche Substanzen, bei denen die Dosen, die Kontraktilität zu steigern, niedriger sind, als die, mit denen die Herzschlagfrequenz steigt.

Gegenstand der Erfindung sind daher neue Aryloxypropanolamine und zwar:

1) Aryloxypropanolamine der allgemeinen Formel I

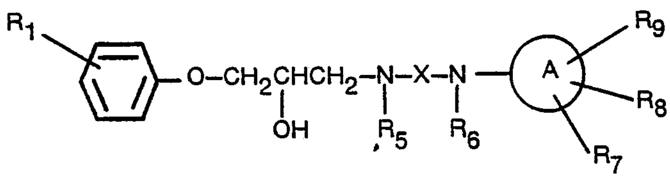

(I)

in welcher

$R_1$ ein Wasserstoffatom, $C_1$—$C_4$-Alkyl, Cyan, Carboxamido, Halogen, Hydroxy, $C_1$—$C_5$-Acyloxy, $C_1$—$C_4$-Alkoxy, Allyloxy, Phenyl-$C_1$—$C_4$-alkoxy,

$R_5$ und $R_6$ gleich oder verschieden sind und ein Wasserstoffatom oder $C_1$—$C_4$-Alkyl,

X eine geradkettige Ethylen- oder Propylengruppe,

A einen mit einem Ring-Kohlenstoff an den Stickstoff gebundenen Rest bedeutet, ausgewählt aus der Gruppe Pyrazol, Pyridin, Indazol, Chinoxalin, Pyrazolon und Purin,

$R_7$, $R_8$, und $R_9$ gleich oder verschieden sind und Wasserstoff, $C_1$—$C_4$-Alkyl oder Allyl bedeuten,

2) Aryloxypropanolamine der allgemeinen Formel I'

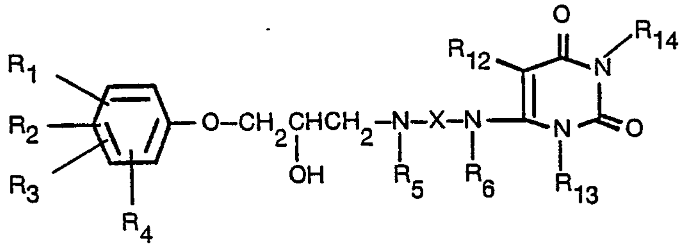

(I'),

in welcher

$R_1$, $R_2$, $R_3$, $R_4$ gleich oder verschieden sind und ein Wasserstoffatom, $C_1$—$C_4$-Alkyl, Cyan, Carboxamido, Halogen, Hydroxy, $C_1$—$C_5$-Acyloxy, $C_1$—$C_4$-Alkoxy, Allyloxy, Phenyl-$C_1$—$C_4$-Alkoxy,

$R_5$ und $R_6$ gleich oder verschieden sind und ein Wasserstoffatom oder $C_1$—$C_4$-Alkyl,

X eine geradkettige Ethylen- oder Propylengruppe,

$R_{12}$ Cyan, $C_1$—$C_4$-Alkyl oder Phenyl

$R_{13}$ $C_1$—$C_4$-Alkyl und
$R_{14}$ $C_1$—$C_4$-Alkyl
bedeuten

3) Aryloxypropanolamine der allgemeinen Formel I''

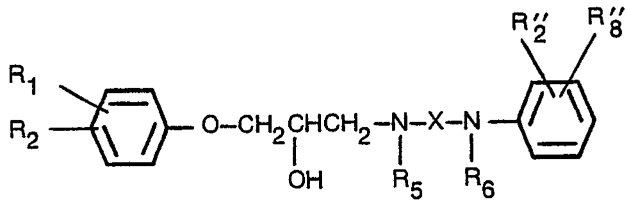

(I''),

in welcher
$R_1$ und $R_2$ gleich oder verschieden sind und ein Wasserstoffatom, $C_1$—$C_5$-Acyloxy, $C_1$—$C_4$-Alkoxy, Cyan, Carboxamido, Halogen, Hydroxy, Allyloxy, Phenyl-$C_1$—$C_4$-alkoxy, $C_1$—$C_4$-Alkyl,
$R_5$ und $R_6$ Wasserstoff,
X eine geradkettige Ethylen- oder Propylengruppe,
$R_7''$ und $R_8''$ gleich oder verschieden sind und Alkyl mit 1—4 C-Atomen
bedeuten, mit der Maßgabe daß $R_1$ und $R_2$ nicht gleich zeitig Wasserstoff sein dürfen und
4) die Verbindungen
1-Phenoxy-3-[2-(1.3.6-trimethyl-pyrimidin-2.4-dion-5-yl-amino)-ethylamino]-propan-2-ol,
1-Phenoxy-3-[2-(1.3.5-trimethyl-pyrimidin-4-on-2-thion-6-yl-amino)-ethylamino]-propan-2-ol und
1-Phenoxy-3-[2-(1.3.5-trimethyl-pyrimidin-2-on-4-thion-6-yl-amino)-ethylamino]-propan-2-ol,
sowie pharmakologisch verträgliche Salze der Verbindungen der obengenannten Formeln.

Da die Verbindungen der obengenannten Formeln asymmetrische Kohlenstoffatome besitzen, sind ferner Gegenstand der Erfindung die optisch aktiven Formen und racemischen Gemische dieser Verbindungen.

Unter einer $C_1$—$C_4$-Alkylgruppe der Reste $R_1$, $R_2$, $R_3$, $R_4$, $R_5$, $R_6$, $R_7$, $R_8$, $R_9$, $R_{12}$, $R_{13}$, $R_{14}$ oder dem $C_1$—$C_4$-Alkylteil komplexer Reste (z.B. Alkoxy) sind geradkettige oder verzweigte Gruppen zu verstehen, wie z.B. Methyl, Ethyl, Propyl, Isopropyl, Butyl, Isobutyl, sek. Butyl oder tert. Butyl. Besonders bevorzugt sind Methyl, Ethyl, Propyl, Isopropyl und Butyl.

Die $C_1$—$C_5$-Acyloxyreste können geradkettig oder verzweigt sein.

Unter Halogen wird im Sinne der Erfindung Fluor, Chlor, Brom und Jod verstanden, insbesondere Fluor, Chlor und Brom.

Ein weiterer Gegenstand der Erfindung sind Verfahren zur Herstellung von Verbindungen der oben erwähnten Folmeln. Die Verbindungen der allgemeinen Formel I werden beispielsweise erhalten, indem man in an sich bekannter Weise entweder

a) eine Verbindung der Formel II

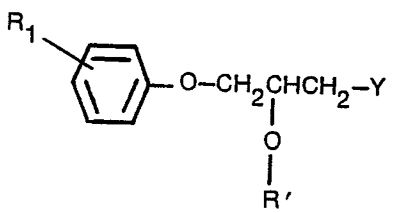

(II)

in welcher
$R_1$ die obengenannte Bedeutung hat,
R' Wasserstoff oder eine Schutzgruppe,
Y eine reaktive Gruppe oder
R' und Y gemeinsam einen Valenzstrich bedeuten,
mit einer Verbindung der Formel III

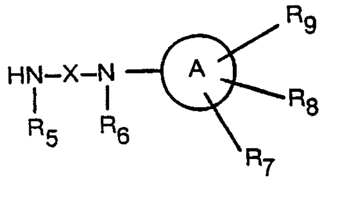

(III)

in welcher
$R_5$, X, $R_6$, A, $R_7$, $R_8$, $R_9$ die obengenannten Bedeutungen haben,
umsetzt oder

b) eine Verbindung der Formel IV

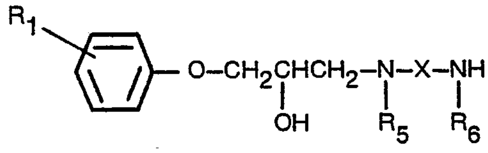

(IV)

in welcher $R_1$, $R_5$, X, $R_6$ die obengenannten Bedeutungen haben, mit einer Verbindung der Formel V

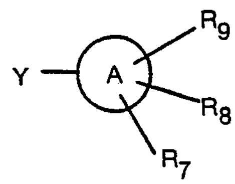

(V)

in welcher
A, $R_7$, $R_8$, $R_9$ die oben angegebenen Bedeutungen haben und
Y eine reaktive Gruppe darstellt,
umsetzt oder

c) eine Verbindung der Formel VI

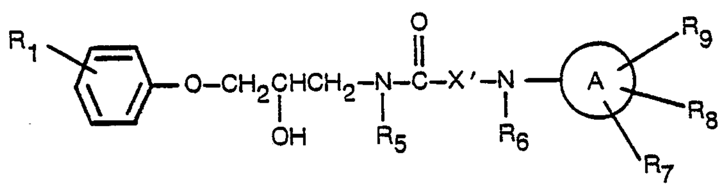

(VI)

in welcher
A, $R_1$, $R_5$, $R_6$, $R_7$, $R_8$, $R_9$ die oben angegebenen Bedeutungen haben und
X' eine geradkettige Methylen- oder Ethylengruppe bedeutet,
reduziert und gegebenenfalls vorhandene Schutzgruppen durch Hydrolyse oder Hydrogenolyse abspaltet.

Die Verbindung der allgemeinen Formel I' erhält man beispielsweise, indem man in an sich bekannter Weise entweder

a) eine Verbindung der Formel II'

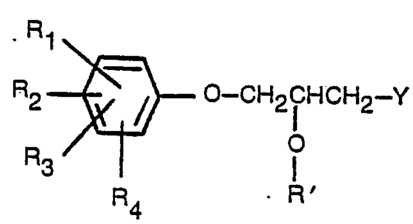

(II')

in welcher
$R_1$, $R_2$, $R_3$, $R_4$ die obengenannten Bedeutungen haben,
R' Wasserstoff oder eine Schutzgruppe,
Y eine reaktive Gruppe oder
R' und Y gemeinsam einen Valenzstrich bedeuten,
mit einer Verbindung der Formel III'

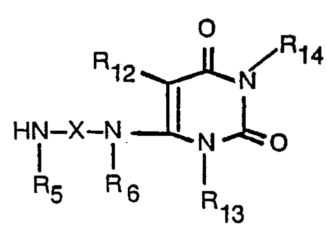

(III')

in welcher

$R_5$, X, $R_6$, $R_{12}$, $R_{13}$, $R_{14}$ die obengenannten Bedeutungen haben,
umsetzt oder

b) eine Verbindung der Formel IV'

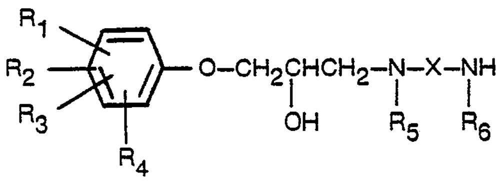

(IV')

in welcher
$R_1$, $R_2$, $R_3$, $R_4$, $R_5$, X, $R_6$ die obengenannten Bedeutungen haben,
mit einer Verbindung der Formel V'

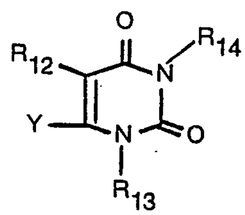

(V')

in welcher
$R_{12}$, $R_{13}$, $R_{14}$ die oben angegebenen Bedeutungen haben und
Y eine reaktive Gruppe darstellt,
umsetzt oder

c) eine Verbindung der Formel VI'

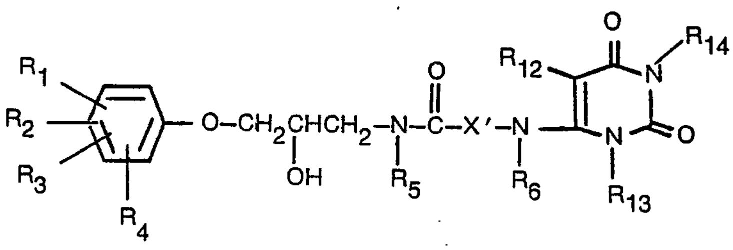

(VI')

in welcher
$R_1$, $R_2$, $R_3$, $R_4$, $R_5$, $R_6$, $R_{12}$, $R_{13}$, $R_{14}$ die oben angegebenen Bedeutung haben und
X' eine geradkettige Methylen- oder Ethylenkette bedeutet,
reduziert und gegebenenfalls vorhandene Schutzgruppen durch Hydrolyse oder Hydrogenolyse abspaltet.

Die Verbindungen der allgemeinen Formel I'' erhält man beispielsweise, indem man in an sich bekannter Weise entweder

a) eine Verbindung der Formel II''

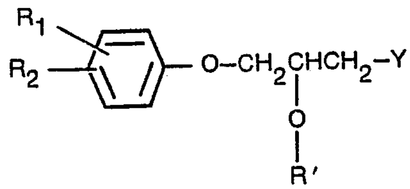

(II'')

in welcher
$R_1$, $R_2$ die obengenannten Bedeutungen haben,
R' Wasserstoff oder eine Schutzgruppe,
Y eine reaktive Gruppe oder
R' und Y gemeinsam einen Valenzstrich bedeuten,
mit einer Verbindung der Formel III''

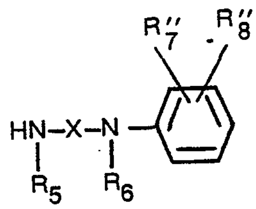

(III'')

in welcher
$R_5$, X, $R_6$, $R_7''$, $R_8''$ die obengennanten Bedeutungen haben,
umsetzt oder

b) eine Verbindung der Formel IV''

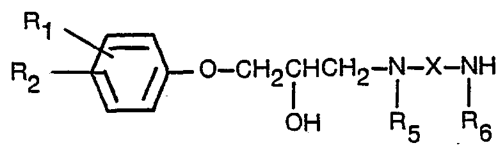

(IV'')

in welcher
$R_1$, $R_2$, $R_5$, X, $R_6$ die obengenannten Bedeutungen haben, mit einer Verbindung der Formel V''

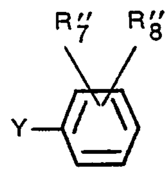

(V'')

in welcher
$R_7''$, $R_8''$ die oben angegebenen Bedeutungen haben und
Y eine reaktive Gruppe darstellt,
umsetzt oder

c) eine Verbindung der Formel VI''

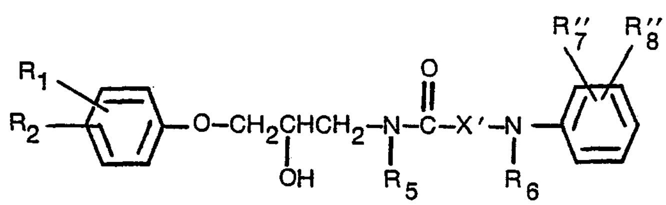

(VI'')

in welcher
$R_1$, $R_2$, $R_5$, $R_6$, $R_7''$, $R_8''$ die oben angegebenen Bedeutungen haben und
X' eine geradkettige Methylen- oder Ethylenkette bedeutet,
reduziert und gegebenenfalls vorhandene Schutzgruppen durch Hydrolyse oder Hydrogenolyse abspaltet.

Die Verbindungen
1-Phenoxy-3-[2-(1.3.6-trimethyl-pyrimidin-2.4-dion-5-yl-amino)-ethylamino]-propan-2-ol,
1-Phenoxy-3-[2-(1.3.5-trimethyl-pyrimidin-4-on-2-thion-6-yl-amino)-ethylamino]-propan-2-ol und
1-Phenoxy-3-[2-(1.3.5-trimethyl-pyrimidin-2-on-4-thion-6-yl-amino)-ethylamino]-propan-2-ol,
erhält man beispielsweise, indem man in an sich bekannter Weise entweder

a) eine Verbindung der Formel II'''

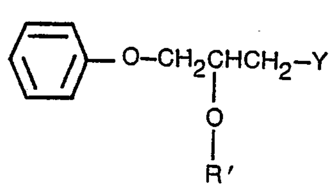

(II''')

in welcher

R' Wasserstoff oder eine Schutzgruppe,
Y eine reaktive Gruppe oder
R' und Y gemeinsam einen Valenzstrich bedeuten,
mit einer Verbindung der Formel III'''

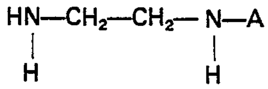 (III''')

in welcher
A einen 1.3.6 - Trimethyl - pyrimidin - 2.4 - dion - 5 - yl -, einen 1.3.5 - Trimethyl - pyrimidin - 4 - on - 2 - thion - 6 - yl -, bzw. einen 1.3.5 - Trimethyl - pyrimidin - 2 - on - 4 - thion - 6 - yl - Rest darstellt,
umsetzt oder

b) eine Verbindung der Formel IV'''

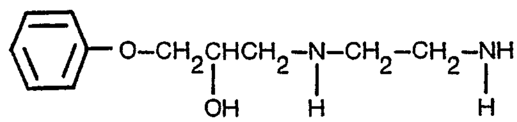 (IV''')

mit einer Verbindung der Formel V'''

Y—A (V''')

in welcher
A die oben angegebene Bedeutung hat und
Y eine reaktive Gruppe darstellt,
umsetzt oder

c) eine Verbindung der Formel VI'''

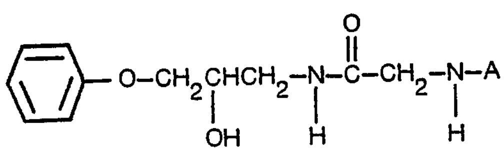 (VI''')

in welcher
A die oben angegebene bedeutung hat,
reduziert und gegebenenfalls vorhandene Schutzgruppen durch Hydrolyse oder Hydrogenolyse abspaltet.

Sämtliche nach den vorstehend genannten Verfahren hergestellten Verbindung können gegebenenfalls in an sich bekannter Weise in andere Verbindungen überführt werden, in an sich bekannter Weise in optisch aktive Formen getrennt und gegebenenfalls durch Umsetzung mit einer anorganischen oder organischen Säure in ein pharmakologisch unbedenkliches Salz überführt werden.

Reaktive Reste Y in Verbindungen der Formeln II, II', II'', II''', V, V', V'' und V''' sind insbesondere Säurereste z.B. von Halogenwasserstoffsäuren und Sulfonsäuren. Besonders bevorzugt sind Chloride, Bromide, Mesyloxy- und Tosyloxyreste.

Zur Herstellung von Verbindungen gemäß der Ansprüchen 1 bis 5 kommen als weitere reaktive Gruppen Y in Verbindungen der Formeln V, V' und V''' auch Methylmercapto- und Methylsulfonylgruppen infrage.

Die Umsetzungen nach Variante a) können, gegebenenfalls unter Verwendung eines inerten Lösungsmittels, bei Raumtemperatur oder durch mäßiges Erwärmen bewirkt werden, wobei es vorteilhaft ist, die Aminkomponente der Formeln III, III', III'' und III''' im Überschuß zu verwenden.

Bei Umsetzungen nach Variante b) kann man die Reaktionskomponente der Formeln IV, IV', IV'' bzw. IV''' und V, V', V'' bzw. V''' in molekularem Verhältnis in einem inerten Lösungsmittel in Gegenwart eines Säureakzeptors umsetzen, beispielsweise in siedendem Toluol in Gegenwart von Kaliumcarbonat.

Nach Variante c) erfolgt die Reduktion der Amidcarbonylgruppe in Verbindungen der Formeln VI, VI', VI'' und VI''' durch komplexe Hydride in einem inerten Lösungsmittel, beispielsweise mit Lithiumaluminiumhydrid in Ether.

Die Anzahl der Kohlenstoffatome des Restes X' muß dabei jeweils um 1 niedriger sein als die des Restes X des herzustellenden Verbindungen.

Als Schutzgruppen finden Verwendung Acylgruppen, vorzugsweise Acetyl und Benzoyl, aber auch die Tetrahydropyranyl- und die Benzylschutzgruppe. Ihre Entfernung erfolgt aus literaturbekannter Weise durch Hydrolyse oder Hydrogenolyse.

Die Herstellung der in den Formeln II, II', II'' und II''' wiedergegebenen Halogenhydrine bzw. 2.3-Epoxypropoxy-derivate kann in bekannter Weise durch Umsetzung entsprechender Phenole mit einer geeigneten reaktiven $C_3$-Komponente in Gegenwart einer Base in wäßrigem oder aprotischem Milieu erfolgen, beispielsweise Umsetzung eines Phenols mit Epichlorhydrin oder Epibromhydrin in wäßriger Natronlauge oder in Dimethylformamid oder Dimethylsulfoxid in Gegenwart von Natriumhydrid.

Die durch die Formeln III, III', III'' und III''' wiedergegebenen N-Aryl- und N-Heteroaryl-alkylendiamine können durch Aminalkylierung erhalten werden, beispielsweise durch Umsetzung eines Anilins oder eines Aminoheterocyclus mit einem Phthalimidoalkylhalogenid in Gegenwart einer Base, worauf man die Verbindungen III, III', III'' und III''' in an sich bekannter Weise durch Hydrazinolyse erhält.

Im Falle, daß A einen Heterocyclus bedeutet, kann auch eine reaktive Verbindung der Formeln V, V' bzw. V''' mit einem Alkylendiamin umgesetzt werden, beispielsweise Umsetzung eines Halogen- oder Methylmercaptopyrimidins mit einem Alkylendiamin, welches in der Regel im Überschuß zur Anwendung kommt.

Verbindungen der Formel III, in welcher A die Bedeutung eines Indazolylrestes hat, können auch durch Anwendung der Bucherer-Lepetit-Reaktion erhalten werden, beispielsweise durch Kochen eines 4-Hydroxy- oder 4-Aminoindazols mit einem Überschuß an Alkylendiaminsulfit in Wasser.

Die neuen Verbindungen der allgemeinen Formeln III, III' und III''' stellen wertvolle Zwischenverbindungen dar zur Herstellung von herz- und kreislaufwirksamen Substanzen, beispielsweise von Verbindungen gemäß Ansprüche 1—5.

Zur Überführung der erfindungsgemäßen Verbindungen in ihre pharmakologisch unbedenklichen Salze setzt man diese, vorzugsweise in einem organischen Lösungsmittel, mit der äquivalenten Menge einer anorganischen oder organischen Säure, z.B. Salzsäure, Bromwasserstoffsäure, Phosphorsäure, Schwefelsäure, Essigsäure, Citronensäure, Maleinsäure, Benzosäure, um.

Die erfindungsgemäßen Verbindungen können aus ihren racemischen Mischungen nach an sich bekannten Methoden über die diastereomeren Salze in die optisch aktiven Formen aufgetrennt werden. Zur Racematspaltung können z.B. Weinsäure, Apfelsäure, Camphersäure, Camphersulfonsäure verwendet werden.

Die erfindungsgemäßen Verbindungen und ihre pharmakologisch verträglichen Salze besitzen wertvolle Wirkungen auf das Herz-Kreislauf-System, insbesondere cardiotone und/oder β-blockierende Wirkungen. Sie eignen sich daher zur Behandlung und Prophylaxe bei Herz- und Kreislauferkrankungen.

Ein weiterer Gegenstand der Erfindung sind daher Arzneimittel, die Verbindungen der allgemeinen Formeln I, I', I'' sowie der Ansprüche 3—5 enthalten.

Zur Herstellung von Arzneimitteln werden die oben genannten Substanzen in an sich bekannter Weise mit geeigneten pharmazeutischen Trägerstoffen, Aroma-, Geschmacks- und Farbstoffen gemischt und beispielsweise als Tabletten oder Dragees ausgeformt oder unter Zugabe entsprechender Hilfsstoffe in Wasser oder Öl, wie z.B. Olivenöl, suspendiert oder gelöst.

Die oben genannten erfindungsgemäßen neuen Substanzen und ihre Salze können in flüssiger oder fester Form enteral oder parenteral appliziert werden. Als Injektionsmedium kommt vorzugsweise Wasser zur Anwendung, welches die bei Injektionslösungen üblichen Zusätze wie Stabilisierungsmittel, Lösungsvermittler oder Puffer enthält. Derartige Zusätze sind z.B. Tartrat- und Citratpuffer, Ethanol, Komplexbildner (wie Ethylendiamintetraessigsäure und deren nicht-toxische Salze), hochmolekulare Polymere (wie flüssiges Polythylenoxyd) zur Viskositätsregulierung. Feste Trägerstoffe sind z.B. Stärke, Lactose, Mannit, Methylcellulose, Talkum, hochdisperse Kieselsäuren, höhermolekulare Fettsäuren (wie Stearinsäure), Gelatine, Agar-Agar, Calciumphosphat, Magnesiumstearat, tierische und pflanzliche Fette und feste hochmolekulare Polymere (wie Polyethylenglykole), für orale Applikation geeignete Zubereitungen können gewünschtenfalls Geschmacks- und Süßstoffe enthalten.

Die Wirksamkeit der erfindungsgemäßen Verbindungen in Vergleich mit repräsentativen Substanzen aus dem Stand der Technik geht aus dem folgenden Versuchsprotokoll hervor:

*Versuchsprotokoll*

Für die Untersuchungen standen Bastard-Hunde beiderlei Geschlechts zur Verfügung, denen in einer vorbereitenden Operation unter aseptischen Bedingungen Katheter in die Arteria und Vena fermoralis sowie durch das Myokard in den linken Ventrikel implantiert worden waren. Frühestens 10 Tage nach dieser Operation, wenn die Tiere sich wieder in einem klinisch gesunden Zustand befanden, wurden die Versuche begonnen.

Während des Vesuches, in dem die Tiere in einem wachen Zustand waren, wurde fortlaufend der arterielle Blutdruck über den Katheter und einen elektromechanischen Druckwandler aufgenommen. Darüberhinaus wurde mittels eines Tipmanometers, der in dem Ventrikelkatheter eingeführt und bis zum Herzen vorgeschoben wurde, der Druck im linken Ventrikel fortlaufend gemessen und daraus die Differentiation nach der Zeit dp/dt max bestimmt. Die Herzfrequenz ($f_{cor}$) wurde durch Auszählen der Herzaktion bei schnellem Papiervorschub zu bestimmten Meßzeitpunkten errechnet.

Die Substanzen wurden in einer Dosis von 0,25 µg/kg pro min über 60 min infundiert, woran sich eine 30-minütige Nachbeobachtung anschloß. Bei einigen Substanzen wurden die Dosen um den Faktor 10 erhöht bzw. die Dosen protrahiert in logarithmischer Dosierung injiziert, um einen möglichst großen Dosisabstand erfassen zu können.

Aus dem Logarithmus, der zu einem bestimmten Zeitpunkt applizierten Dosis und der Wirkung auf dp/dt max bzw. Frequenz ($f_{cor}$) wurde eine lineare Regression berechnet. Daraus ließen sich die Dosen $DE_{+30\%}$ und $DE_{+50\%}$ (µg-kg) kalkulieren, die eine Erhöhung der Ausgangswerte von dp/dt $_{max}$ bzw. $f_{cor}$ um 30 bzw. 50% bewirkten (Beispiel siehe Tab. 1).

Die Ergebnisse der Vergleichsverbindungen sowie der eigenen Substanzen sind in Tabelle 2 zusammengefaßt. Aus ihr ist zu entnehmen, daß die Vergleichsverbindung A, 1 - Phenoxy - 3 - [2 - (2,6 - dimethyl - phenylamino) - ethylamino] - propan - 2 - ol (Beispiel 7 aus DE—A—28 44 497, VEB Dresden), zwar eine gute cardiotone Wirkung (Anstieg von dp/dt max) hat, in gleicher Dosis wird jedoch auch die Frequenz gesteigert, was prinzipiell unerwünscht ist, weil es das Herz zu stark belastet.

Als Maß für die Qualität der Substanzen wurden zunächst die Dosen gesucht, die die Herzkraft (dp/dt max) und die Herz-frequenz ($f_{cor}$) um 30 bzw. 50% steigern. Bildet man einen Quotienten aus $ED_{+30\%}$ $f_{cor}$ durch $ED_{+30\%}$ dp/dt, sind besonders die Substanzen wertvoll, die einen hohen Ouotienten aufweisen, da bei diesen Substanzen, auf die Dosis bezogen, die Steigerung der Herzkraft stärker ist, als die der Frequenz; je höher der Quotient also, desto besser die Substanz. Dagegen können generell niedrige Dosen nicht als Kriterium einer besonders günstigen Wirkung angesehen werden; es kommt auf das Verhältnis von Wirkung auf Herzfrequenz zu Kontraktilität an.

Die Vergleichssubstanz B, 1 - Phenoxy - 3 - [2 - (1.3 - dimethyl - pyrimidin - 2.4 - dion - 6 - yl - amino) - ethylamino] - propan - 2 - ol (Beispiel 1 aus DE—A—28 19 629, Cassella), weist keine cardiotone Wirkung auf.

TABELLE 1

Prüfung ausgewählter Substanzen auf carbiotone Wirkung an wachen Hunden

$\bar{p}a$ = mittlerer arterieller Blutdruck (mm Hg)

$f_{cor}$ = Herzfrequenz (Schläge/min)

dp/dt = Druckanstiegsgeschwindigkeit im linken Ventrikel (mm Hg/sec)

$DE_{+30\%}$ bzw. $DE_{+50\%}$ = Dosis, die eine Erhöhung der Ausgangswerte um 30% bzw. 50% bewirkt (µg/kg).

| Substanz | Meßwert | Ausgangs-wert | ...min nach Infusionsbeginn ...µg/kg der Prüfsubstanz | | | | | | $DE_{+30\%}$ | $DE_{+50\%}$ | Regressions-gleichung |
|---|---|---|---|---|---|---|---|---|---|---|---|
| | | | 5<br>1,25 | 10<br>2,5 | 15<br>3,75 | 30<br>7,5 | 45<br>11,25 | 60<br>15,0 | | | |
| A* | $\bar{p}a$ | 96 | 97 | 99 | 98 | 98 | 105 | 105 | | | |
| | $f_{cor}$ | 92 | 93 | 115 | 108 | 123 | 141 | 149 | 4,5 | 10,9 | y = 53 log x + 95,5 |
| | dp/dt | 2,15 | 2,05 | 2,48 | 2,60 | 2,90 | 3,68 | 3,48 | 4,4 | 8,8 | y = 67 log x + 86,5 |
| Substanz aus | $\bar{p}a$ | 98 | 97 | 106 | 100 | 105 | 106 | 102 | | | |
| | $f_{cor}$ | 55 | 57 | 77 | 63 | 65 | 85 | 103 | 3,81 | 8,55 | y = 57 log x + 96,9 |
| Bsp. 14 | dp/dt | 2,30 | 3,20 | 3,30 | 3,40 | 3,80 | 4,40 | 4,80 | 1,55 | 2,74 | y = 81 log x + 114,4 |

* 1-Phenoxy-3-[2-(2,6-dimethylphenylamino)-ethylamino]-propan-2-ol (Beispiel 7 aus DE-A-28 44 497, VEB Dresden)

Die Beispiele sind lediglich zur Verdeutlichung des Auswerteverfahrens aufgeführt.

TABELLE 2

In analoger Weise, wie in Tabelle 1 dargestellt, ermittelte Daten für weitere Verbindungen:

| Bsp. Nr. | DE $_{+30\%}$ (µg/kg) dp/dt | $f_{cor}$ | $\frac{f_{cor}}{dp/dt}$ | DE $_{+50\%}$ (µg/kg) dp/dt | $f_{cor}$ | $\frac{f_{cor}}{dp/dt}$ |
|---|---|---|---|---|---|---|
| A* | 4,4 | 4,5 | 1,0 | 8,8 | 10,9 | 1,2 |
| B** | >150 | >150 | – | >150 | >150 | – |
| 14 | 1,55 | 3,81 | 2,46 | 2,74 | 8,55 | 3,15 |
| 10 | 22,6 | >150 | >6,6 | 47,1 | >150 | >3,2 |
| 20 | 45,4 | 311 | 6,9 | 155 | 669 | 4,3 |
| 38 | 21,2 | 70,8 | 3,3 | 85,1 | 742 | 8,7 |
| 1 | 9,7 | 18,7 | 1,9 | 36,8 | 75,5 | 2,1 |
| 32 | 236 | 2734 | 11,6 | 589 | 8979 | 15,2 |
| 28 | 72,7 | 160 | 2,20 | 110 | 348 | 3,16 |
| 18 | 27,7 | 82,8 | 2,99 | 39,0 | 135 | 3,46 |

* 1-Phenoxy-3-[2-(2,6-dimethylphenylamino)-ethylamino]-propan-2-ol. (Beispiel 7 aus DE–OS 28 44 497, VEB Dresden).

** 1-Phenoxy-3-[2-(1.3-dimethyl-pyrimidin-2.4-dion-6-yl-amino)-ethylamino]-propan-2-ol. (Beispiel 1 aus DE–OS 28 19 629, Cassella).

Neben den in den Beispielen aufgeführten Verbindungen werden noch als bevorzugt genannt:
1-Phenoxy-3-[2-(1.3-dimethyl-5-isopropyl-pyrimidin-2.4-dion-6-yl-amino)-ethylamino]-propan-2-ol
1-Phenoxy-3-[2-(1.5-dimethyl-3-ethyl-pyrimidin-2.4-dion-6-yl-amino)-ethylamino]-propan-2-ol
1-Phenoxy-3-[2-(1.3.5-trimethyl-pyrimidin-2.4-dion-6-yl-N-methylamino)-ethylamino]-propan-2-ol
1-Phenoxy-3-[2-(3.5-dimethyl-pyrazol-4-yl-amino)-ethylamino]-propan-2-ol

Die Erfindung wird durch die folgenden Beispiele näher erläutert. Sie zeigen einige der zahlreichen möglichen Verfahrensvarianten auf, die zur Synthese der erfindungsgemäßen Verbindungen verwendet werden können.

Beispiel 1

1-Phenoxy-3-[2-(1.2.4-trimethyl-5-pyrazolon-3-ylamino)-ethylamino]-propan-2-ol-oxalat

3.8 g Phenyl-glycidether werden mit 8.8 g 3-(2-Aminoethylamino)-1.2.4-trimethyl-5-pyrazolon 22 Stunden bei 70°C gerührt. Man löst in Methylenchlorid und reinigt säulenchromatographisch an Kieselgel mit den Laufmitteln Methylenchlorid-Methanol 9:1 und Methylenchlorid-Methanol-Triethylamin 20:2:1.

Ausbeute: 5.2 g (61% d.Th.) Öl.

Aus Essigester wird durch Zusatz der äquivalenten Menge Oxalsäure das Oxalat gefällt und dieses aus Ethanol unter Verwendung von Aktivkohle umkristallisiert: farblose Kristalle, Fp. 141—142°C (Z).

Das als Ausgangsverbindung verwendete 3-(2-Amino-ethylamino)-1.2.4-trimethyl-5-pyrazolon kann auf folgendem Wege erhalten werden:

Methylmalonsäurediethylester und N.N'-Dimethylhydrazin wereden in Gegenwart von Natriummethylat im Methanol 170 Stunden unter Stickstoff am Rückfluss erhitzt. Man erhält *3-Hydroxy-1.2.4-trimethyl-5-pyrazolon* als farblose Kristalle, Fp. 87—89°C (aus Essigester).

Aus der vorstehenden Verbindung erhält man durch Umsetzung mit Phosphoroxitrichlorid (3.5 Stunden Rückfluss) über das 3.5-Dichlor-1.2.4-trimethyl-pyrazoliumsalz nach Zerlegung in wässriger Natronlauge und Extraktion mit Methylenchlorid *3-Chlor-1.2.4-trimethyl-5-pyrazolon,* Fp. 37—38°C.

Dieses wird mit einem Überschuss (15 x) Ethylendiamin umgesetzt (22 Stunden Rückfluss). Die Aufarbeitung erfolgt durch Einengen, Entsalzen in methonolischer Lösung durch den Ionenaustauscher IRA—400 (OH-Form) und Reinigen über Kieselgel mit dem Laufmittel Methylenchlorid-ammoniakal. Methanol 8:2 Man erhält in einer Ausbeute von 81% d.Th. *3-(2-Amino-ethylamino)-1.2.4-trimethyl-5-pyrazolon* als gelbliches Öl.

Beispiel 2

1-Phenoxy-3-[2-(1.3.5-trimethyl-pyrazol-4-yl-amino)-ethylamino]-propan-2-ol

3.8 g Phenyl-glycidether werden mit 8.4 g 4-(2-Amino-ethylamino)-1.3.5-trimethyl-pyrazol 48 Stunden bei Raumtemperatur stehengelassen. Man löst in Methylenchlorid, schüttelt mit Wasser aus, trocknet und reinigt chromatographisch über Kieselgel mit dem Laufmittel Methylenchlorid-ammoniakal. Methanol 92:8. Nach Einengen der reinen Fraktionen erhält man 4.7 g (59% d.Th.) eines Öls.

Man löst dieses in Essigester, gibt 1.7 g Fumarsäure zu, reibt den zuerst schmierigen Niederschlag mit Isopropanol an und kristallisiert aus Ethanol um: farblose Kristalle, Fp. 124°C (Z.).

Das als Ausgangsverbindung verwendete 4-(2-Amino-ethylamino)-1.3.5-trimethyl-pyrazol kann wie folgt erhalten werden:

Man setzt 4-Amino-1.3.5-trimethyl-pyrazol mit N-(2-Bromethyl)-phthalimid in Gegenwart von Kaliumcarbonat um (in Acetonitril 16 Stunden Rückfluss) und erhält in guter Ausbeute *4-(2-Phthalimido-ethylamino)-1.3.5-pyrazol*, gelbliche Kristalle, Fp. 122—123°C (aus Ethanol), worauf man durch Hydrazinolyse *4-(2-Amino-ethylamino)-1.3.5-trimethyl-pyrazol* als Öl erhält.

Beispiel 3

1-Phenoxy-3-[2-(3-methyl-2-pyridylamino)-ethylamino]-propan-2-ol

wird aus Phenyl-glycidether und 2-(2-Amino-ethylamino)-3-methyl-pyridin erhalten: farblose Kristalle (33% d.Th.), Fp. 80—81°C (Essigester).

Das als Ausgangsverbindung verwendete *2-(2-Amino-ethylamino)-3-methyl-pyridin* wird aus 2-Brom-3-methyl-pyridin und einem Überschuss (15 x) Ethylendiamin (20 Stunden Rückfluss) erhalten und durch Destillation gereinigt: farbloses Öl (65% d.Th.) $Kp_{0,01}$ 81—83°C.

Beispiel 4

1-Phenoxy-3-[2-(1.3.5-trimethyl-pyrimidin-2.4-dion-6-yl-amino)-ethylamino]-propan-2-ol

3.4 g Phenyl-glycidether und 9.6 g 4-(2-Amino-ethylamino)-1.3.5-trimethyl-pyrimidin-2.6(1H,3H)-dion werden bei 70°C ca. 24 Stunden gerührt. Man löst in Methanol und trennt das Gemisch säulenchromatographisch unter Verwendung des schwach sauren Kationenaustauschers Amberlite CG 50 II pract. (Serva). Vorbehandlung der Säule mit ca. 1.5 Liter methanolischer 0.1 M Triethylammoniumacetatlösung.

Laufmittel: 0.1 M methanolische Triethylammoniumacetatlösung. Die Fraktionen der reinen Substanz werden eingeengt, worauf man mit 2 N Natronlauge alkalisch stellt, mit Methylenchlorid extrahiert, den Extrakt trocknet und einengt. Das erhaltene helle Öl wird durch Anreiben mit Ether kristallin. Durch Umkristallisieren aus Essigester werden 3.6 g (44% d.Th) farblose Kristalle, Fp. 104—106°C, erhalten.

Dans als Ausgangsverbindung verwendete *4-(2-Amino-ethylamino)-1.3.5-trimethyl-pyrimidin-2.6-(1H,3H)-dion* lässt sich wie folgt erhalten:

In 120 ml Ethylendiamin trägt man in kleinen Portionen bei 10—15°C 22.6 g 4-Chlor-1.3.5.-trimethyl-pyrimidin-2.6(1H,3H)-dion ein und rührt noch 2 Stunden bei Raumtemperatur. Am Rotavapor wird schonend eingeengt (Badtemperatur < 35—40°C), das erhaltene Produkt in 100 ml Methanol gelöst und durch Amberlite IRA—400 (OH-Form) laufen gelassen. Durch schonendes Einengen erhält man nach Anreiben mit Ether 18.8 g (74% d.Th.) farblose Kristalle, Fp. 81—82°C.

Beispiel 5

Analog Beispiel 4 erhält man durch Umsetzung von 4 - Propoxyphenyl - glycidether mit 4 - (2 - Amino - ethylamino) - 1.3.5 - trimethyl - pyrimidin - 2.6(1H,3H) - dion *1 - (4 - Propoxy - phenoxy) - 3 - [2 - (1.3.5 - trimethyl - primidin - 2.4 - dion - 6 - yl - amino) - ethylamino] - propan - 2 - ol* farblose Kristalle (32% d.Th.), Fp. 121—123°C (Methanol).

Beispiel 6

1-(4-Benzyloxyphenoxy)-3-[2-(2.6-dimethylphenylamino)-ethylamino]-propan-2-ol

5.12 g 4-Benzyloxyphenyl-glycidether und 6.56 g N-(2.6-Dimethylphenyl)-etheylendiamin erhitzt man unter Rühren 20 Stunden auf 70°C. Die Aufarbeitung erfolgt durch Ionenaustauschchromatographie mit Amberlite CG 50 II pract. (Serva) wie in Beispiel 4 angegeben.

Ausbeute: 6.8 g (81% d.Th.) helles, viscoses Öl.

Beispiel 7

1-(4-Propoxyphenoxy)-3-[2-(2.6-dimethylphenylamino)-ethylamino]-propan-2-ol

4.15 g 4-Propoxyphenyl-glycidether und 6.6 g N-(2.6-Dimethylphenyl)-ethylendiamin lässt man 3 Tage bei Raumtemperatur stehen und trennt das Gemisch durch Ionenaustauschchromatographie wie für Beispiel 4 angegeben.

Ausbeute: 4.3 g (58% d.Th.) farblose Kristalle,
Fp. 51—54°C; neutrales Fumarat, Fp. 168—169°C (Ethanol).

Beispiel 8

1-(3.4-Dimethoxyphenoxy)-3-[2-(1.3.5-trimethyl-pyrimidin-2.4-dion-6-yl-amino)-ethylamino]-propan-2-ol

4.2 g (3.4-Dimethoxyphenyl-glycidether und 8.5 g 4-(2-Amino-ethylamino)-1.3.5-trimethyl-pyrimidin-2.6(1H,3H)-dion lässt man mit 5 ml Dimethylformamid 48 Stunden bei Raumtemperatur stehen.

Man löst in Methylenchlorid, schüttelt fünfmal mit Wasser aus, extrahiert die organische Phase mit 1 N Essigsäure, stellt die wässrige Phase durch Zugabe von verd. Natronlauge alkalisch und extrahiert diese mit Methylenchlorid. Nach Trocknen wird über Kieselgel chromatographiert mit dem Laufmittel Methylenchlorid- Methanol-ammoniakal. Methanol (gesättigt) 20:1:1.

Der nach Einengen der reinen Fraktionen erhaltene Rückstand wird aus Essigester umkristallisiert.

Ausbeute: 2.0 g (24% d.Th.) farblose Kristalle,
Fp. 103—104°C.

Beispiel 9

In analoger Weise, wie in Beispiel 8 beschrieben, erhält man:

a) *1 - (4 - Methoxyphenoxy) - 3 - [2 - (1.3.5 - trimethyl - pyrimidin - 2.4 - dion - 6 - yl - amino) - ethylamino] - propan - 2 - ol*

29% d.Th., Fp. 115—117°C (Essigester), aus 4-Methoxyphenyl-glycidether und 4-(2-Amino-ethylamino)-1.3.5-trimethyl-pyrimidin-2.6(1H,3H)-dion

b) *1-(4-Benzyloxyphenoxy)-3-[2-(1.3.5-trimethyl-pyrimidin-2.4-dion-6-yl-amino)-ethylamino]-propan-2-ol*

30% d.Th., farbloses Öl, aus 4-Benzyloxyphenyl-glycidether und 4-(2-Amino-ethylamino)-1.3.5-trimethyl-pyrimidin-2.6(1H,3H)-dion

c) *1 - (3.4 - Dibenzyloxy - phenoxy) - 3 - [2 - (1.3.5 - trimethyl - pyrimidin - 2.4 - dion - 6 - yl - amino) - ethylamino] - propan - 2 - ol*

32% d.Th., farbloses Öl, aus 3.4 - Dibenzyloxyphenyl - glycidether und 4 - (2 - Amino - ethylamino) - 1.3.5 - trimethyl - pyrimidin - 2.6(1H,3H) - dion

Beispiel 10

1-Phenoxy-3-[3-(1.3.5-trimethyl-pyrimidin-2.4-dion-6-yl-amino)-propylamino]-propan-2-ol- neutrales Fumarat

3.8 g Phenyl-glycidether werden mit 10 g 4-(3-Amino-propylamino)-1.3.5-trimethyl-pyrimidin-2.6-(1H,3H)-dion 20 Stunden bei Raumtemperatur stehengelassen, worauf man an Kieselgel mit dem Laufmittel Methylenchlorid-ammoniakal. Methanol 9:1 chromatographiert. Das nach Einengen der reinen Fraktionen erhaltene Öl (5.5 g) wird in Essigester mit 1.7 g Fumarsäure versetzt und das erhaltene Salz aus Ethanol umkristallisiert.

Ausbeute: 2.5 g (23% d.Th.) farblose Kristalle, Fp. 132—133°C.

Das als Ausgangsverbindung verwendete *4-(3-Amino-propylamino)-1.3.5-trimethyl-pyrimidin-2.6(1H,3h)-dion,* farbloses Öl, kann in guter Ausbeute durch Umsetzung von 4-Chlor-1.3.5-trimethyl-pyrimidin-2.6(1H,3H)-dion mit einem Überschuss (15 x) 1.3-Diaminopropan bei 10—15°C und schonender Aufarbeitung erhaten werden.

Beispiel 11

1-Phenoxy-3-[2-(1.3.6-trimethyl-pyrimidin-2.4-dion-5-yl-amino)-ethylamino]-propan-2-ol

2.43 g Phenyl-glycidether und 5.15 g 5-(2-Amino-ethylamino)-1.3.6-trimethyl-pyrimidin-2.4(1H,3H)-dion lässt man 24 Stunden bei Raumtemperatur stehen, worauf das Gemisch über Kieselgel, Laufmittel Methylenchlorid-ammoniakal. Methanol 95:5, getrennt wird. Das durch Einengen der reinen Fraktionen erhaltene gelbliche Öl lässt sich mit Essigester anreiben. Durch Umkristallisieren aus Essigester werden. 2.55 g (43% d.Th.) farblose Kristalle, Fp. 92—94°C, erhalten.

Das als Ausgangsverbindung verwendete 5-(2-Amino-ethylamino)-1.3.6-trimethyl-pyrimidin-2.4-(1H,3H)-dion kann durch folgende Reaktion erhalten werden:

1. *5-(2-Phthalimido-ethylamino)-1.3.6-trimethyl-pyrimidin-2.4(1H,3H)-dion*

24.7 g 5-Amino-1.3.6-trimethyl-pyrimidin-2.4(1H,3H)-dion, 44.5 g N-(2-Bromethyl)-phthalamid und 30.2 g Kaliumcarbonat werden in 450 ml Acetonitril 4 Tage im Autoklaven bei 110—130°C gerührt. Man saugt die anorgan. Salze ab, engt ein und reinigt über Kieselgel mit dem Laufmittel Essigester.

Ausbeute: 13.7 g (27% d.Th.) farblose Kristalle, Fp. 178—180°C.

2. *5-(2-Amino-ethylamino)-1.3.6-trimethyl-pyrimidin-2.4(1H,3H)-dion*

11.7 g der vorstehenden Phthalimidoverbindung werden mit 1.92 ml Hydrazinhydrat in 140 ml Ethanol 2 Stunden gekocht, worauf man ansäuert und 1 Stunde weitererhitzt. Nach Erkalten saugt man

Phthalhydrazid ab, engt ein, löst in Methanol und entsalzt die Lösung durch den Ionenaustauscher Amberlite IRA—400 (OH-Form). Zur weiteren Reinigung engt man ein und chromatographiert über eine Kieselgelsäule mit dem Laufmittel Methylenchlorid-ammoniakal. Methanol 9:1.

Ausbeute: 5.2 g (71% d.Th.) gelbliches Öl.

Beispiel 12

1-Phenoxy-3-[2-(4-indazolylamino)-ethylamino]-propan-2-ol-benzoat

3.0 g Phenyl-glycidether und 7.0 g 4-(2-Amino-ethylamino)indazol werden in 7 ml Dimethylformamid durch Erwärmen gelöst, worauf man 20 Stunden bei Raumtemperatur stehen lässt. Man löst in Methylenchlorid, schüttelt zehnmal mit Wasser aus, trocknet mit Natriumsulfat, behandelt die Lösung mit Bleicherde, engt ein, löst in Essigester und gibt 3.0 g Benzoesäure zu. Das Erhaltene Salz wird aus Isopropanol unter Zusatz von Aktivkohle und Bleicherde umkristallisiert.

Ausbeute: 3.9 g (43% d.Th.) farblose Kristalle, Fp. 155—156°C.

Das als Ausgangsverbindung verwendete *4-(2-Amino-ethylamino)-indazol* kann aus 4-Hydroxy-indazol und einem Überschuss (10 x) Ethylendiamin-sulfit mit einer Ausbeute von 43% d.Th. erhalten werden (in wässriger Lösing 1 Stunde Rückfluss): hellbeige Kristalle, Fp. 138—140°C (Methylenchlorid).

Beispiel 13

1-(4-Hydroxyphenoxy-3-[2-(2.6-dimethylphenylamino)-ethylamino]-propan-2-ol- neutrales Fumarat

6.8 g 1-(4-Benzyloxyphenoxy)-3-[2-(2.6-dimethylphenylamino)-ethylamino]-propan-2-ol wird in 100 ml Methanol in Gegenwart von 1 g Palladium-Kohle (10 proz.) hydriert. Die nach Absaugen und Einengen erhaltene zähe Masse wird in 300 ml Essigester mit Fumarsäure heiss gerührt. Das erhaltene Salz kristallisiert man aus Wasser unter Zusatz von Aktivkohle um.

Ausbeute: 2.7 g (43% d.Th.) fast farblose Kristalle, Fp. 193—195°C.

Beispiel 14

1-(4-Hydroxyphenoxy)-3-[2-(1.3.5-trimethyl-pyrimidin-2.4-dion-6-yl-amino)-ethylamino]-propan-2-ol-neutrales Fumarat

farblose Kristalle, Fp. 213—215°C (Wasser) wird in analoger Weise, wie für Beispiel 13 beschrieben, durch Hydrierung von 1 - (4 - Benzyloxyphenoxy) - 3 - [2 - (1.3.5 - trimethyl - pyrimidin - 2.4 - dion - 6 - yl - amino) - ethylamino] - propan - 2 - ol erhalten.

Beispiel 15

1-(4-Methoxyphenoxy)-3-[2-(1.3.5-trimethyl-pyrimidin-2.4-dion-6-yl-amino)-ethylamino]-propan-2-ol

4.8 g 1-(4-Methoxyphenoxy)-3-(2-amino-ethylamino)-propan-2-ol, 3.8 g 4-Chlor-1.3.5-trimethyl-pyrimidin-2.6(1H,3H)-dion und 4.2 g Kaliumcarbonat werden in 50 ml Toluol 4 Tage unter Rückfluss gerührt. Man engt im Vakuum ein, rührt den Rückstand mit Methylenchlorid, saugt die anorgan. Salze ab, engt erneut ein und reibt mit einem Gemisch Toluol-Essigester 3:1 an. Durch Umkristallisieren aus Essigester unter Verwendung von Aktivkohle und Bleicherde werden 2.4 g (31% d.Th.) farblose Kristalle, Fp. 115—117°C, erhalten.

Das als Ausgangsverbindung verwendete *1-(4-Methoxyphenoxy)-3-(2-amino-ethylamino)-propan-2-ol* erhält man in guter Ausbeute aus 4-Methoxyphenyl-glycidether und einem Überschuss Ethylendiamin (1 Stunde Rückfluss) als helles, erstarrendes Öl.

Beispiel 16

1-(4-Fluorphenoxy-3-[2-(2.6-dimethyl-phenylamino)-ethylamino]-propan-2-ol

3.36 g 4-Fluorphenyl-glycidether werden mit 6.6 g N-(2.6-Dimethylphenyl)-ethylendiamin 2 Tage bei Raumtemperatur stehengelasen. Man trennt das Gemisch durch Chromatographie an Kieselgel mit dem Laufmittel Essigester-Methanol-Triethylamin 100:10:1 und erhält nach Einengen der reinen Fraktionen 5.0 g der Titelverbindung als zäles Öl.

Nach Lösen in Ether wird durch Zugabe von 2.5 g Benzoesäure das Benzoat gefällt und dieses aus Isopropanol umkristallisiert.

Ausbeute: 5.2 g (57% d.Th.) farblose Kristalle, Fp. 105—106°C.

Beispiel 17

1-(2.5-Dichlorphenoxy-3-[2-(2.6-dimethyl-phenylamino)-ethylamino]-propan-2-ol

wird analog Beispiel 16 aug 2.5-Dichlorphenyl-glycidether und N-(2.6-Dimethylphenyl)-ethylendiamin erhalten, wobei bereits die Base kristallisiert.

Ausbeute: 4.2 g (55% d.Th.) farblose Kristalle, Fp. 66—68°C (Ether-Ligroin).

Beispiel 18

1-(4-Hydroxyphenoxy-3-[3-(2.6-dimethyl-phenylamino]·-propylamino]-propan-2-ol-neutrales Oxalat

2.5 g 1-(4-Benzyloxy-phenoxy)-3-[3-(2.6-dimethyl-phenylamino)-propylamino]-propan-2-ol werden in 200 ml Methanol in Gegenwart von 0.25 g Palladium-Kohle (10 proz.) hydriert. Das nach Absaugen und

Einengen erhaltene farblose Öl (2.0 g) löst man in Ethanol. Man fällt durch Zugabe einer ethanolischen Oxalsäurelösung das Oxalat und kristallisiert dieses 2 x aus Ethanol um.

Ausbeute: 1.0 g (45% d.Th.) farblose Kristalle, Fp. 198—199°C.

Das als Ausgangsverbindung verwendete 1-(4-Benzyloxy-phenoxy)-3-[3-(2.6-dimethyl-phenylamino)-propylamino]-propan-2-ol kann durch folgende Reaktionen erhalten werden:

1. *N-(3-Phthalimido-propyl)-2.6-dimethylanilin*

50 g N-(3)-Brompropyl)-phthalimid und 50 g 2.6-Dimethylanilin werden 5 Stunden bei 100°C gerührt. Nach Zusatz von Ether saugt man ab und erhält 69 g des Hydrobromids (Fp. 226—228°C). Die Base wird aus Wasser mit konz. Ammoniak freigesetzt.

Ausbeute: 48 g (83% d.Th.) farblose Kristalle, Fp. 97—98°C.

2. *N-(2.6-Dimethylphenyl)-1.3-diamino-propan*

Durch Hydrazinolyse der Phthalimido-Verbindung und Entsalzen mittels Ionenaustauscher, wird ein farbloses Öl erhalten.

3. *1-(4-Benzyloxy-phenoxy)-3-[3-(2.6-dimethyl-phenylamino)-propylamino]-propan-2-ol*

4.23 g 4-Benzyloxyphenyl-glycidether, 5.9 g N-(2.6-Dimethylphenyl)-1.3-diamino-propan und 5 ml Isopropanol werden 24 Stunden bei Raumtemperatur gerührt.

Die Aufarbeitung erfolgt durch Chromatographie an Kieselgel.

Beispiel 19

1-(3.4-Dihydroxyphenoxy-3-[2-(2.6-dimethyl-phenylamino)-ethylamino]-propan-2-ol benzoat

7.9 g 1-(3.4-Dibenzyloxyphenoxy)-3-[2-(2.6-dimethyl-phenylamino)-ethylamino]-propan-2-ol werden in 100 ml Methanol in Gegenwart von 0.5 g Palladium-Kohle (10 proz.) hydriert. Der nach Absaugen und Einengen erhaltene Rückstand wird in Essigester gelöst, worauf man durch Zugabe einer Lösung von 2.5 g Benzoesäure in Essigester das Benzoat zur Kristallisation bringt. Dieses wird abgesaugt und aus Essigester unter Verwendung von Bleicherde umkristallisiert.

Ausbeute: 3.3 g (47% d.Th.) fast farblose Kristalle, Fp. 139—142°C.

Die Ausgangsverbindung wird wie folgt erhalten: *1-(3.4-Dibenzyloxyphenoxy)-3-[2-(2.6-dimethyl-phenylamino)-ethylamino]-propan-2-ol.* Ein Gemisch aus 6.6 g N-(2.6-Dimethylphenyl)-ethylenediamin und 7.2 g 3.4-Dibenzyloxyphenyl-glycidether wird 2 Tage bei Raumtemperatur stehengelassen. Man trennt über eine Kieselgelsäule mit dem Laufmittel Essigester-Methanol-Triethylamin 100:10:1 und erhält nach Einengen der reinen Fraktionen 7.9 g (75% d.Th.) eines farblosen Öles.

Beispiel 20

1-(4-Fluorphenoxy-3-[2-(1.3.5-trimethyl-pyrimidin-2.4-dion-6-ylamino)-ethylamino]-propan-2-ol-hydrogenfumarat

3.36 g 4-Fluorphenyl-glycidether und 8.5 g 4-(2-Amino-ethylamino-1.3.5-trimethyl-pyrimidin-2.6(1H,3H)-dion werden in 10 ml Dimethylformamid gelöst und 2 Tage bei Raumtemperatur stehenge lassen. Man giesst in 100 ml Wasser, extrahiert mit Methylenchlorid, trocknet und engt ein. Das erhaltene zähe Öl wird durch Säulenchromatographie über Kieselgel mit dem Laufmittel Methylenchlorid-ammoniakgesättigtes Methanol 20:1 getrennt. Das durch Einengen der reinen Fraktionen erhaltene Produkt wird in 20 ml Essigester gelöst, worauf man 1.8 g Fumarsäure, gelöst in Ethanol, zusetzt. Nach Absaugen wird das erhaltene kristalline Produkt aus Ethanol unter Zusatz von 0.9 g Fumarsäure und unter Verwendung von Aktivkohle umkristallisiert.

Ausbeute: 5.2 g (52% d.Th.) farblose Kristalle, Fp. 163—167°C.

Beispiel 21

1-(2-Cyanphenoxy)-3-[2-(1.3.5-trimethyl-pyrimidin-2.4-dion-6-ylamino)-ethylamino]-propan-2-ol-hydrogenfumarat

Umsetzung und Aufarbeitung analog wie für Beispiel 20 beschrieben aus 2-Cyanphenyl-glycidether und 4-(2-Amino-ethylamino)-1.3.5-trimethyl-pyrimidin-2.6(1H,3H)-dion.

37% d.Th., farblose Kristalle, Fp. 164—167°C (Ethanol unter Zusatz von etwas Fumarsäure).

Beispiel 22

1-(3.4-Dihydroxyphenoxy-3-[2-(1.3.5-trimethyl-pyrimidin-2.4-dion-6-ylamino)-ethylamino]-propan-2-ol-neutrales Fumarat

10.4 g 1-(3.4-Dibenzyloxyphenoxy)-3-[2-(1.3.5-trimethyl-pyrimidin-2.4-dion-6-ylamino)-ethylamino]-propan-2-ol werden in 100 ml Methanol in Gegenwart von 1 g Palladium-Kohle (10 proz.) hydriert. Der nach Absaugen und Einengen erhaltene Rückstand wird in wenig Ethanol gelöst, worauf man eine ethanolische Lösung von 1 g Fumarsäure zusetzt. Nach erfolgter Kristallisation wird abgesaugt und aus Ethanol-Wasser unter Zusatz von 1 g Hydrochinon und Verwendung von Aktivkohle umkristallisiert.

Ausbeute: 3.5 g (43% d.Th.) fast farblose Kristalle, Fp. 195—197°C. (Z).

Die verwendete Ausgangsverbindung wird wie folgt erhalten: *1 - (3.4 - Dibenzyloxyphenoxy) - 3 - [2 - (1.3.5 - trimethyl - pyrimidin - 2.4 - dion - 6 - ylamino) - ethylamino] - propan - 2 - ol* 8.5 g 3.4-Dibenzyloxyphenyl-ylglycidether und 7.2 g 4-(2-Amino-ethylamino)-1.3.5-trimethyl-pyrimidin-2.6(1H,3H)-dion, gelöst in 8 ml Dimethylformamid werden 2 Tage bei Raumtemperatur stehengelassen. Man löst in

Methylenchlorid, schüttelt mit viel Wasser aus, trocknet und reinigt durch Chromatographie an Kieselgel mit dem Laufmittel Essigester-Methanol-Methylamin 100:10:2.

Ausbeute: 10.4 g (77% d.Th.) farbloses Öl.

Beispiel 23

1-Phenoxy-3-[2-(1.3-dimethyl-5-ethyl-pyrimidin-2.4-dion-6-ylamino)-ethylamino]-propan-2-ol-hydrogenfumarat

3.3 g Phenyl-glycidether und 10 g 4-(2-Amino-ethylamino)-1.3-dimethyl-5-ethyl-pyrimidin-2.6(1H,3H)-dion werden gemischt und 24 Stunden bei Raumtemperatur stehengelassen. Man löst in Methylenchlorid und reinigt chromatographisch an Kieselgel mit dem Laufmittel Methylenchlorid-Methanol-$NH_3$-gesättigt 9:1. Durch Einengen der reinen Fraktionen werden 5.5 g eines gelblichen Öles erhalten. Dieses wird in Essigester gelöst, worauf man eine Lösung von 1.7 Fumarsäure in Essigester zugibt. Nach erfolgter Kristallisation wird abgesaugt und aus Ethanol umkristallisiert.

Ausbeute: 2.8 g (26% d.Th.) farblose Kristalle, Fp. 138—139°C (Balsenbildung).

Die verwendete Ausgangsverbindung kann wie folgt erhalten werden: *4 - (2 - Amino - ethylamino) - 1.3 - dimethyl - 5 - ethyl - pyrimidine - 2.6(1H,3H) - dion* 30 g 4-Chlor-1.3-dimethyl-5-ethyl-pyrimidin-2.6-(1H,3H)-dion werden in kleinen Portionen bei 10—15°C unter Rühren in 100 ml Ethylendiamin eingetragen. Man rührt noch 2 Stunden bei Raumtemperatur, entfernt überschüssiges Ethylendiamin schonend am Rotavapor, löst in Methanol, entsalzt durch Amberlite IRA—400 (OH-Form), engt ein und reinigt das erhaltene gelbliche Öl chromatographisch an einer Kieselgelsäule mit den Laufmitteln 1. Methylenchlorid-Methanol 9:1, 2. Methylenchlorid-Methanol-$NH_3$-gesättigt (9:1). Die reinen Fraktionen werden eingeengt und man erhält 13.2 g (39% d.Th.) einer zähen, farbloses Masse.

Beispiel 24

1-Phenoxy-3-[2-(1.3-dimethyl-5-n-butyl-pyrimidin-2.4-dion-6-ylamino)-ethylamino]-propan-2-ol-sesquihydrogenfumarat

5.5 g 4-Chlor-1.3-dimethyl-5n-butyl-pyrimidin-2.6(1H,3H)-dion und 5.0 g 1-Phenoxy-3-(2-amino-ethylamino)-propan-2-ol werden in 20 ml Dimethylformamid 4 Tage bei 80°C gerührt. Man engt ein, löst den Rückstand in Methylenchlorid, schüttelt mit Wasser aus, trocknet und reinigt chromatographisch über eine Kieselgelsäule mit dem Laufmittel Methylenchlorid-Methanol 9:1. Durch Einengen der reinen Fraktionen werden 4.4 g eines gelblichen Öls erhalten. Dieses wird in Essigester gelöst und mit einer Lösung von 1.3 g Fumarsäure in Essigester versetzt. Nach erfolgter Kristallisation saugt man ab und wäscht mit Essigester nach. Ausbeute: 2.4 g (17% d.Th.) farblose Kristalle, 76°C Sintern, Fp. 78—80°C (unter Blasenbildung).

Beispiel 25

1-Phenoxy-3-[2-(1.3-dimethyl-5-phenyl-pyrimidin-2.4-dion-6-ylamino)-ethylamino]-propan-2-ol-hydrogenfumarat

5.0 g 4-Chlor-1.3-dimethyl-5-phenyl-pyrimidin-2.6(1H,3H)-dion, 4.2 g 1-Phenoxy-3-(2-amino-ethylamino)-propan-2-ol und 3.5 ml N-Ethyl-diisopropylamin werden in 20 ml Acetonitril 48 Stunden unter Rückfluß erwärmt. Man engt im Vacuum ein, nimmt den Rückstand in Methylenchlorid auf, schüttelt die Lösung mit 2 N Natronlauge, trocknet und trennt säulenchromatographisch an Kieselgel mit dem Laufmittel Methylenchlorid-Methanol-$NH_3$-gesättigtes Methanol 40:1:1.

Das durch Einengen der reinen Fraktionen erhaltene Öl (5.3 g) wird in 10 ml Essigester gelöst, worauf man mit einer Lösung von 2 g Fumarsäure in 20 ml Ethanol versetzt. Nach erfolgter Kristallisation wird abgesaugt und mit Essigester nachgewaschen.

Ausbeut: 6.7 g (62% d.Th.) farblose Kristalle, Fp. 181—183°C.

Beispiel 26

1-Phenoxy-3-[2-(1.3-dimethyl-5-cyan-pyrimidin-2.4-dion-6-ylamino)-ethylamino]-propan-2-ol-hydrochlorid

Unter Kühlung werden 5 g 4-Chlor-5-cyan-1.3-dimethyl-pyrimidin-2.6(1H,3H)-dion in kleinen Portionen in eine Lösung von 5.26 g 1-Phenoxy-3-(2-amino-ethylamino)-propan-2-ol in 20 ml Dimethylformamid eingetragen. Man rührt 2 Stunden bei Raumtemperatur, versetzt mit 60 ml Isopropanol, saugt ab und wäscht mit Isopropanol nach.

Ausbeute: 8.7 g (85% d.Th.) farblose Kristalle, Fp. 202—204°C.

Beispiel 27

1-Phenoxy-3-[2-(1.3-diethyl-5-methyl-pyrimidin-2.4-dion-6-ylamino)-ethylamino]-propan-2-ol-hydrogenfumarat

3.8 g Phenyl-glycidether und 9.8 g 4-(2-Amino-ethylamino)-5-methyl-1.3-diethyl-pyrimidin-2.6(1H,3H)-dion werden in 10 ml Dimethylformamid gelöst und 24 Stunden bei Raumtemperatur stehengelassen. Man gießt in Wasser, extrahiert mit Methylenchlorid, trocknet und chromatographiert an Kieselgel mit dem Laufmittel Methylenchlorid-Methanol 9:1. Das nach Einengen der reinen Fraktionen erhaltene gelbiiche Öl (6.8 g) wird in Essigester gelöst worauf man eine Lösung von 2.1 g Fumarsäure in Essigester zusetzt. Der erhaltene kristalline Niederschlag wird abgesaugt und mit Essigester gewaschen.

Ausbeute: 5.4 g (42% d.Th.) farblose Kristalle, Fp. 141—142°C (unter Blasenbildung)

Beispiel 28

1-Phenoxy-3-[2-(1.3.5-trimethyl-pyrimidin-2.4-dion-6-ylamino)-ethyl-N-methylamino]-propan-2-ol-hydrogenfumarat

4.7 g 4-Chlor-1.3.5-trimethyl-pyrimidin-2.6(1H,3H)-dion, 5.6 g 1-Phenoxy-3-(2-aminoethyl-N-methyl-amino)-propan-2-ol werden in Gegenwart von 3.2 g N-Ethyl-diisopropylamin in 30 ml Toluol 24 Stunden bei 90°C gerührt. Man engt ein, nimmt in Essigester auf, schüttelt mit Wasser aus, trocknet und chromatographiert an Kieselgel mit dem Laufmittel Essigester-Methanol 10:1. Durch Einengen der reinen Fraktionen werden 4.0 g eines Öls erhalten welches man in Essigester löst. Man setzt eine Lösung von 1.2 g Fumarsäure in Essigester-Ethanol zu, saugt nach erfolgter Kristallisation ab und kristallisiert aus Ethanol um.

Ausbeute: 2.9 g (24% d.Th.) farblose Kristalle, Fp. 166—167°C.

Die verwendete Ausgangsverbindung kann durch folgende Reaktionen erhalten werden:

1. *1-Phenoxy-3-(2-phthalimidoethyl-N-methyl-amino)-propan-2-ol*
aus Phenyl-glycidether und N-(2-Methylamino-ethyl)-phthalimid als hellgelbes Öl.

2. *1-Phenoxy-3-(2-aminoethyl-N-methyl-amino)-propan-2-ol*
durch Hydrazinolyse und anschließender Entsalzung mit Amberlite IRA—400 (OH-Form): hellgelbes Öl.

Beispiel 29

1-Phenoxy-3-[2-(1.3.5-trimethyl-pyrimidin-2.4-dion-6-yl-N-methylamino)-ethyl-N'-methyl-amino]-propan-2-ol

4.7 g 4-Chlor-1.3.5-trimethyl-pyrimidin-2.6 (1H,3H)-dion und 6.0 g 1-Phenoxy-3-(2-methylamino-ethyl-N-methylamino)-propan-2-ol ($Kp_{0.01}$ 138—142°C, aus Phenyl-glycidether und N.N'-Dimethyl-ethylen diamin) werden in 10 ml Pyridin 72 Stunden bei 80°C gerührt. Man Löst in Methylenchlorid, wäscht mit Wasser, trocknet und chromatographiert an Kieselgel mit dem Laufmittel Methylenchlorid-Methanol 20:1. Durch Einengen der reinen Fraktionen werden 3.0 g (31% d.Th.) eines fast farblosen Öls erhalten.

Beispiel 30

1-Phenoxy-3-[2-(1.3.5-trimethyl-pyrimidin-4on-2-thion-6-ylamino)-ethylamino]-propan-2-ol-hydrogen-fumarat

4.3 g 4-Chlor-1.3.5-trimethyl-pyrimidin-6(1H)-on-2(3H)-thion werden mit 4.4 g 1-Phenoxy-3-(2-amino-ethylamino)-propan-2-ol gemischt und 24 Stunden bei 60°C gerührt. Man reinigt chromatographisch an Kieselgel mit dem Laufmittel Essigester-Methanol 8:2.

Durch Einengen der reinen Fraktionen erhält man ein Öl (2.9 g) welches man in Essigester löst und mit einer Lösung von 0.9 g Fumarsäure in Essigester-Ethanol versetzt. Man saugt ab und kristallisiert aus Ethanol um.

Ausbeute: 2.0 g (19% d.Th.) blaßgelbe Kristalle, Fp. 143—144°C (unter Blasenbildung)

Das als Ausgangsverbindung verwendete *4-Chlor-1.3.5-trimethyl-pyrimidin-6(1H)-on-2(3H)-thion,* blaßgelbe Kristalle, Fp 128—129°C (Isopropanol), erhält man in guter Ausbeute aus 4-Hydroxy- 1.3.5-pyrimidin-6(1H)-on-2(3H)-thion durch Umsetzung mit Phosphoroxitrichlorid (2 Stunden Rückfluß).

Beispiel 31

1-Phenoxy-3-[2-(1.3.5-trimethyl-pyrimidin-2-on-4-thion-6-lyamino)-ethylamino]-propan-2-ol-hydrogenfumarat

5.0 g 1-Phenoxy-3-(2-amino-ethylamino)-propan-2-ol und 5.2 g 4-Methylmercapto-1.3.5-trimethyl-pyrimidin-2(3H)-on-6(1H)-thion werden in 150 ml Isopropanol 22 Stunden am Rückfluß erhitzt. Man reinigt chromatographisch wie bei Beispiel 23 angegeben. Das erhaltene leicht gelbliche Öl (7.5 g) loest man in Essigester. Man fügt eine Lösung von 2.3 g Fumarsäure, gelöst in Essigester+Ethanol zu, saugt nach erfolgter Kristallisation an und kristallisiert aus Ethanol um.

Ausbeute: 5.8 g (49% d.Th.) blaßgelbe Kristalle, Fp. 151—153°C (unter Blasenbildung).

Das als Ausgangsverbindung verwendete *4-Methylmercapto-1.3.5-trimethyl-pyrimidin-2(3H)-on-6(1H)thion,* gelbe Kristalle, Fp. 93—94°C (Essigester), wird mit guter Ausbeute durch Methylieren von 4-Mercapto-1.3.5-trimethyl-pyrimidin-2(3H)-on-6(1H)-thion erhalten.

Beispiel 32

1-Phenoxy-3-[2-(3-methyl-chinoxalin-2-ylamino)-ethylamino]-propan-2-ol

5.6 g Phenyl-glycidether und 15 g 2-(2-Amino-ethylamino)-3-methyl-chinoxalin-werden in wenig Dimethylformamid gelöst und 32 Stunden auf 40°C erwärmt. Man gießt in Wasser, extrahiert mit Methylenchlorid, trocknet und reinigt chromatographisch an Kieselgel wie für Beispiel 24 angegeben.

Ausbeute: 4.2 g (33% d.Th) viskoses Öl.

Aus Essigester wird durch Zugabe von Fumarsäure ein Salz gefällt un dieses aus Ethanol umkristallisiert:

Farblose Kristalle, Fp. 152—153°C (neutrales Fumarat-Hydrat).

Das als Ausgansverbindung verwendete *2-(2-Amino-ethylamino)-3-methyl-chinoxalin* wird durch Umsetzung von 2-Chlor-3-methyl-chinoxalin mit einem Überschuß (15 x) Ethylendiamin und Entsalzung durch Ionenaustauscher (OH—Form) erhalten: farblose Kristalle, Fp. 60—61°C (Xylol).

Beispiel 33

1-(4-Fluorphenoxy)-3-[2-(indazol-4-ylamino)-ethylamino]-propan-2-ol-benzoat

3.36 g 4-Fluorphenyl-glycidether und 7.0 g 4-(2-Amino-ethylamino)-indazol werden in 10 ml Dimethylformamid gelöst und 2 Tage bei Raumtemperatur stehen gelassen. Man gießt in Wasser, extrahiert mit methylenchlorid, trocknet und reinigt chromatographisch an Kieselgel wie bei Beispiel 23 angegeben. Der Rückstand der reinen Fraktionen wird in Essigester aufgenommen worauf man mit 2 g Benzoesäure versetzt, absaugt und aus Isopropanol unter Verwendung von Aktivkohle umkristallisiert.

Ausbeute: 5.6 g (60% d.Th.) farblose Kristalle, Fp. 157—159°C.

Beispiel 34

1-(4-Hydroxyphenoxy)-3-[2-(indazol-4-ylamino)-ethylamino]-propan-2-ol

5.0 g 1-(4-Benzyloxyphenoxy)-3-[2-(indazol-4-ylamino)-ethylamino]-propan-2-ol werden in Gegenwart von 0.5 g Palladium-Kohle (10%) in 100 ml Methanol hydriert. Nach Absaugen wird das Filtrat eingeengt, der Rückstand mit Ethanol angerieben. Man löst heiß in Dimethylformamid, behandelt mit Aktivkohle, versetzt mit der doppelten Menge Wasser, saugt ab und wäscht mit Wasser, zuletzt mit Ethanol.

Ausbeute: 3.0 g (76% d.Th.) blass rosastichige Kristalle, Fp. 216—218°C.

Das als Ausgangsverbindung verwendete *1-(4-Benzyloxyphenoxy)-3-[2-(indazol-4-ylamino)-ethylamino]-propan-2-ol* kann wie folgt erhalten werden:

5.1 g 4-Benzyloxyphenyl-glycidether und 7.0 g 4-(2-Amino-ethylamino)-indazol werden in 10 ml Dimethylformamid gelöst und 2 Tage bei Raumtemperatur stehen gelassen. Man digeriert durch Zugabe von 20 ml Methanol, saugt ab und wäscht mit Methanol nach. Ausbeute: 5.0 g (58% d.Th.) farblose Kristalle, Fp. 151—153°C.

Beispiel 35

1-Phenoxy-3-[3-(indazol-4-ylamino)-propylamino]-propan-2-ol-benzoat

3.0 g Phenyl-glycidether und 7.5 g 4-(3-Amino-propylamino)-indazol werden in 10 ml Dimethylformamid bei 60°C gelöst, dann 1 Tag bei Raumtemperatur stehen gelassen. Man gießt in 100 ml Wasser, extrahiert mit Methylenchlorid, trocknet und reinigt chromatographisch an Kieselgel wie für Beispiel 20 angegeben.

Der zähe Rückstand der reinen Fraktionen (5.3 g, 78% d.Th.) wird in wenig Essigester gelöst, worauf man 2 g Benzoesäure zusetzt, absaugt und aus Ethanol unter Verwendung von Aktivkohle umkristallisiert.

Ausbeute: 2.8 g (30% d.Th.) farblose Kristalle, Fp. 162—164°C.

Die verwendete Ausgangsverbindung kann wie folgt erhalten werden *4-(3-Amino-propylamino)-indazol* In einer Lösung von 74 g 1.3-Diamino-propan in 150 ml Wasser leitet man Schwefeldioxid bis pH 7 ein, worauf man 13.4 g 4-Hydroxy-indazol zugibt und 3 Stunden auf 100°C erhitzt. Durch Zusatz der doppelten Menge Methanol werden Salze ausgefällt, von denen man absaugt. Das Filtrat wird eingeengt, der ölige Rückstand durch konz. Ammoniak alkalisch gestellt, worauf man mit Methylenchlorid extrahiert. Durch Einengen des Extraktes werden 7.8 g (41% d.Th.) graue Kristalle, Fp. 154—163°C erhalten.

Beispiel 36

1-Phenoxy-3-[2-(indazol-5-ylamino)-ethylamino]-propan-2-ol

3.0 g Phenyl-glycidether und 7.0 g 5-(2-Amino-ethylamino)-indazol werden in 10 ml Dimethylformamid durch kurzes Erwärmen auf 70°C gelöst, dann 24 Stunden bei Raumtemperatur stehen gelassen. Man schüttelt mit 400 ml Methylenchlorid-Methanol 8:2 und 200 ml Wasser, engt die organische Phase ein und reinigt den Rückstand durch Säulenchromatographie an Kieselgel mit den Laufmitteln Methylen-chlorid-Methanol 9:1 und Methylenchlorid-ammoniakgesättigtes Methanol 9:1. Der Rückstand der reinen Fraktionen wird in 50 ml Essigester 1 Stunde am Rückfluß zum Sieden erhitzt, worauf man nach Erkalten absaugt und aus Isopropanol unter Verwendung von Aktivkohle umkristallisiert.

Ausbeute: 1.7 g (26% d.Th.) fast farblose Kristalle, 137°C Sintern, Fp. 141—144°C.

Die verwendete Ausgangsverbindung kann wie folgt erhalten werden: *5-(2-Amino-ethylamino)-indazol*

Ein Gemisch aus 12.0 g 5-Amino-indazol, 146 g Ethylendiaminsulfit, 146 ml Wasser und 10 ml n-Propanol wird 24 Stunden unter Rühren auf 100°C erwärmt. Nach Erkalten wird mit 500 ml Methanol verdünnt. Nach Absaugen der ausgefallenen Salze wird das Filtrat eingeengt, der Rückstand durch Zugabe von konz. Ammoniak alkalisch gestellt. Man saugt ab, wäscht mit Wasser nach und erhält 9.4 g Kristalle, Fp. 150—165°C.

Beispiel 37

1-Phenoxy-3-[2-(indazol-7-ylamino)-ethylamino]-propan-2-ol

3.0 g Phenyl-glycidether und 7.0 g 7-(2-Amino-ethylamino)-indazol werden in 20 ml Isopropanol 10 Stunden bei 70°C gerührt. Man engt ein und reinigt chromatographisch an einer Kieselgelsäule wie bei Beispiel 23 angegeben.

Der Rückstand der reinen Fraktionen wird mit Essigester digeriert und dann aus Essigester unter Verwendung von Aktivkohle und Bleicherde umkristallisiert.

Ausbeute: 2.3 g (35% d.Th.) farblose Kristalle, Fp. 117—119°C.

Die verwendete Ausgangsverbindung *7-(2-Amino-ethylamino)-indazol*, 67% d.Th., farblose Kristalle, Fp. 166—168°C (Essigester-Isopropanol) wird durch Umsetzung von 7-Hydroxy-indazol mit einem Überschuß Ethylendiaminsulfit in Wasser (3 Stunden 110°C) erhalten.

Beispiel 38

1-Phenoxy-3-[2-(5-methyl-indazol-4-ylamino)-ethylamino]-propan-2-ol-benzoat

3.0 g Phenyl-glycidether und 6.0 g 5-Methyl-4-(2-amino-ethylamino)-indazol werden in 12 ml Dimethylformamid gelöst und 2 Tage bei Raumtemperatur stehen gelassen. Man nimmt in wenig Methylenchlorid auf, schüttelt dreimal mit der 10 fachen Menge Wasser aus, trocknet die Methylenchloridlösung und trennt säulenchromatographisch an Kieselgel mit dem Laufmittel Methylenchlorid-Methanol-Triethylamin 100:10:1. Durch Einengen der reinen Fraktionen werden 5.2 g eines dunklen Öls erhalten. Man löst dieses in wenig Essigester, versetzt mit 2.5 g Benzosäure, saugt ab und kristallisiert aus Essigester unter Verwendung von Aktivkohle und Bleicherde um. Ausbeute: 3.3 g (36% d.Th.) farblose Kristalle, Fp. 97—103°C.

Die verwendete Ausgangsverbindung kann über folgende Stufen erhalten werden:

1. *4-Amino-5-methyl-indazol,* Fp. 197—200°C (93% d.Th.), durch Reduktion ($H_2$[Pd/C] in Methanol) von 4-Nitro-5-methyl-indazol,

2. *5-Methyl-4-(2-amino-ethylamino)-indazol,* (31% d.Th.) Fp. 153—155°C (Wasser), durch Umsetzung von 4-Amino-5-methyl-indazol mit einem Überschuß Ethylendiaminsulfit in Ethylenglycol-Wasser (1:1), 24 Stunden 110°C.

Beispiel 39

1-Phenoxy-3-[2-(purin-6-ylamino)-ethylamino]-propan-2-ol

4.5 g Phenyl-glycidether und 14.2 g 6-(2-Amino-ethylamino)-purin werden in 100 ml Dimethylformamid 10 Stunden bei 50°C gerührt. Man nimmt in Methylenchlorid auf, schüttelt mit Wasser aus, trocknet und reinigt chromatographisch an einer Kieselgelsäule wie für Beispiel 24 angegeben. Das durch Einengen der reinen Fraktionen erhaltene Produkt wird aus Ethanol umkristallisiert Ausbeute: 2.3 g (23% d.Th.) farblose Kristalle, Fp, 169—170°C (Blasenbildung).

Das als Ausgangsverbindung verwendete *6-(2-Amino-ethylamino)-purin* wird in guter Ausbeute durch Umsetzung von 6-Chlor-purin mit einem Überschuß Ethylendiamin erhalten.

**Patentansprüche**

1. Aryloxypropanolamine der allgemeinen Formel I

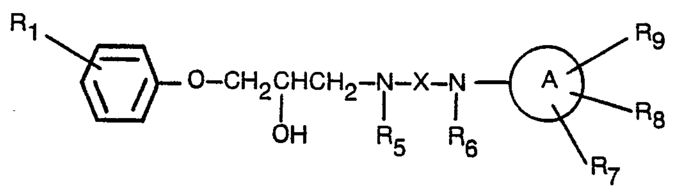

(I)

in welcher

$R_1$ ein Wasserstoffatom, $C_1$—$C_4$-Alkyl, Cyan, Carboxamido, Halogen, Hydroxy, $C_1$—$C_5$-Acyloxy, $C_1$—$C_4$-Alkoxy, Allyloxy, Phenyl-$C_1$—$C_4$-alkoxy,

$R_5$ und $R_6$ gleich oder verschieden sind und ein Wasserstoffatom oder $C_1$—$C_4$-Alkyl,

X eine geradkettige Ethylen- oder Propylengruppe,

A einen mit einem Ring-Kohlenstoff an den Stickstoff gebundenen Rest bedeutet, ausgewählt aus der Gruppe Pyrazol, Pyridin, Indazol, Chinoxalin, Pyrazolon und Purin,

$R_7$, $R_8$ und $R_9$ gleich oder verschieden sind und Wasserstoff, $C_1$—$C_4$-Alkyl oder Allyl bedeuten, deren optisch aktive Formen, racemische Gemische sowie deren pharmakologisch verträgliche Salze.

2. Aryloxypropanolamine der allgemeinen Formel I'

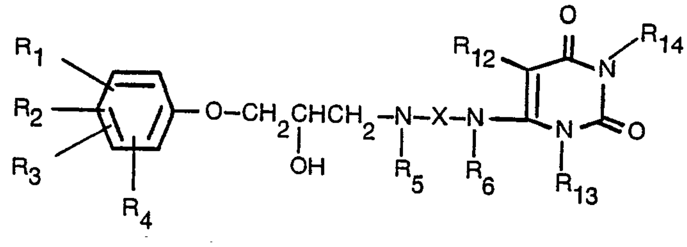

(I'),

in welcher

$R_1$, $R_2$, $R_3$, $R_4$ gleich oder verschieden sind und ein Wasserstoffatom, $C_1$—$C_4$-Alkyl, Cyan, Carboxamido, Halogen, Hydroxy, $C_1$—$C_5$-Acyloxy, $C_1$—$C_4$-Alkoxy, Allyloxy, Phenyl-$C_1$—$C_4$-Alkoxy,

$R_5$ und $R_6$ gleich oder verschieden sind und ein Wasserstoffatom oder $C_1$—$C_4$-Alkyl,

X eine geradkettige Ethylen- oder Propylengruppe,

$R_{12}$ Cyan, $C_1$—$C_4$-Alkyl oder Phenyl

$R_{13}$ $C_1$—$C_4$-Alkyl und

$R_{14}$ $C_1$—$C_4$-Alkyl bedeuten, deren optisch aktive Formen, racemische Gemische sowie deren pharmakologisch verträgliche Salze.

3. 1-Phenoxy-3-[2-(1.3.6-trimethyl-pyrimidin-2.4-dion-5-yl-amino)-ethylamino]-propan-2-ol.

4. 1-Phenoxy-3-[2-(1.3.5-trimethyl-pyrimidin-4-on-2-thion-6-yl-amino)-ethylamino]-propan-2-ol, sowie dessen Fumarat.

5. 1-Phenoxy-3-[2-(1.3.5-trimethyl-pyrimidin-2-on-4-thion-6-yl-amino)-ethylamino]-propan-2-ol, sowie dessen Fumarat.

6. Aryloxypropanolamine der allgemeinen Formel I''

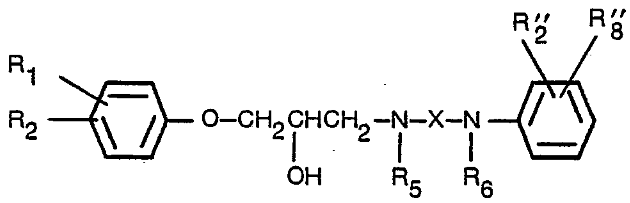

(I''),

in welcher

$R_1$ und $R_2$ gleich oder verschieden sind und ein Wasserstoffatom, $C_1$—$C_5$-Acyloxy, $C_1$—$C_4$-Alkoxy, Cyan, Carboxamido, Halogen, Hydroxy, Allyloxy, Phenyl-$C_1$—$C_4$-alkoxy, $C_1$—$C_4$-Alkyl,

$R_5$ und $R_6$ Wasserstoff,

X eine geradkettige Ethylen- oder Propylengruppe,

$R_7''$ und $R_8''$ gleich oder verschieden sind und Alkyl mit 1—4 C—Atomen bedeuten, mit der Maßgabe, daß $R_1$ und $R_2$ nicht gleichzeitig Wasserstoff sein dürfen, deren optisch aktive Formen, racemische Gemische sowie deren pharmakologisch verträgliche Salze.

7. Verfahren zur Herstellung von Aryloxypropanolaminen der allgemeinen Formel I

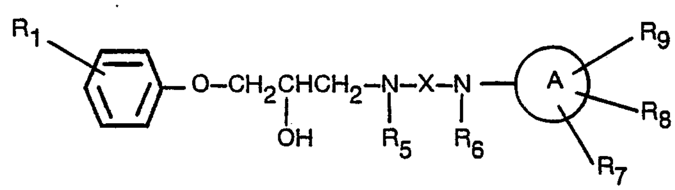

(I)

in welcher

$R_1$ ein Wasserstoffatom, $C_1$—$C_4$-Alkyl, Cyan, Carboxamido, Halogen, Hydroxy, $C_1$—$C_5$-Acyloxy, $C_1$—$C_4$-Alkoxy, Allyloxy, Phenyl-$C_1$—$C_4$-alkoxy.

$R_5$ und $R_6$ gleich oder verschieden sind und ein Wasserstoffatom oder $C_1$—$C_4$-Alkyl,

X eine geradkettige Ethylen- oder Propylengruppe,

A einen mit einem Ring-Kohlenstoff an den Stickstoff gebundenen Rest bedeutet, ausgewählt aus der Gruppe Pyrazol, Pyridin, Indazol, Chinoxalin, Pyrazolon und Purin,

$R_7$, $R_8$ und $R_9$ gleich oder verschieden sind und Wasserstoff, $C_1$—$C_4$-Alkyl oder Allyl bedeuten, deren optisch aktive Formen, racemische Gemische sowie deren pharmakologisch verträgliche Salze, dadurch gekennzeichnet, daß man in an sich bekannter Weise entweder

a) eine Verbindung der Formel II

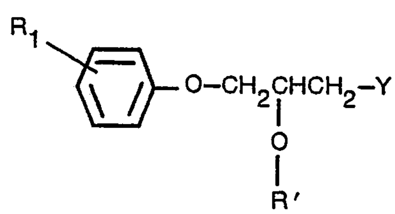

(II)

in welcher

$R_1$ die obengenannte Bedeutung hat,

R' Wasserstoff oder eine Schutzgruppe,

Y eine reaktive Gruppe oder

R' und Y gemeinsam einen Valenzstrich bedeuten, mit einer Verbindung der Formel III

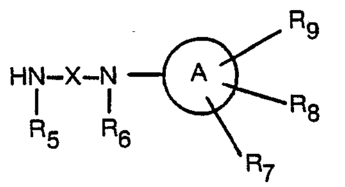

(III)

in welcher
$R_5$, X, $R_6$, A, $R_7$, $R_8$, $R_9$ die obengenannten Bedeutungen haben, umsetzt oder
b) eine Verbindung der Formel IV

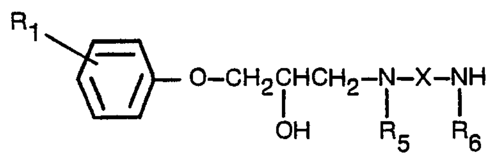

(IV)

in welcher
$R_1$, $R_5$, X, $R_6$ die obengenannten Bedeutungen haben, mit einer Verbindung der Formel V

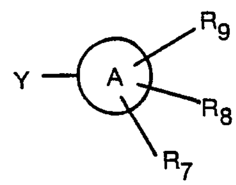

(V)

in welcher
A, $R_7$, $R_8$, $R_9$ die oben angegebenen Bedeutungen haben und
Y eine reaktive Gruppe darstellt, umsetzt oder
c) eine Verbindung der Formel VI

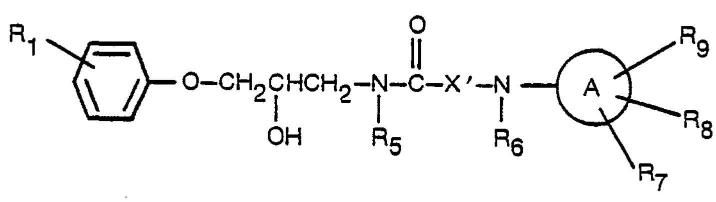

(VI)

in welcher
A, $R_1'$, $R_5$, $R_6$, $R_7$, $R_8$, $R_9$ die oben angegebenen Bedeutungen haben und
X' eine geradkettige Methylen- oder Ethylengruppe bedeutet,
reduziert und gegebenenfalls vorhandene Schutzgruppen durch Hydrolyse oder Hydrogenolyse abspaltet,
und gegebenenfalls anschließend die erhaltenen Verbindungender allgemeinen Formel I in andere Verbindungen der allgemeinen Formel I in an sich bekannter Weise überführt,
gegebenenfalls ein racemisches Gemisch von Verbindungen der allgemeinen Formel I in an sich bekannter Weise in optisch aktive Formen trennt
und gegebenenfalls das resultierende Aryloxypropanolamin der allgemeinen Formel I durch Umsetzung mit einer anorganischen oder organischen Säure in ein pharmakologisch unbedenkliches Salz überführt.
8. Verfahren zu Herstellung von Aryloxypropanolaminen der allgemeinen Formel I'

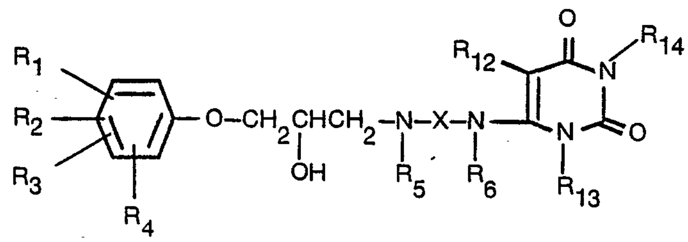

(I'),

in welcher

$R_1$, $R_2$, $R_3$, $R_4$ gleich oder verschieden sind und ein Wasserstoffatom, $C_1$—$C_4$-Alkyl, Cyan, Carboxamido, Halogen, Hydroxy, $C_1$—$C_5$-Acyloxy, $C_1$—$C_4$-Alkoxy, Allyloxy, Phenyl-$C_1$—$C_4$-Alkoxy,

$R_5$ und $R_6$ gleich oder verschieden sind und ein Wasserstoffatom oder $C_1$—$C_4$-Alkyl,

X eine geradkettige Ethylen- oder Propylengruppe,

$R_{12}$ Cyan, $C_1$—$C_4$-Alkyl oder Phenyl

$R_{13}$ $C_1$—$C_4$-Alkyl und

$R_{14}$ $C_1$—$C_4$-Alkyl bedeuten, deren optisch aktive Formen, racemische Gemische sowie deren pharmakologisch verträgliche Salze, dadurch gekennzeichnet, daß man in an sich bekannter Weise entweder

a) eine Verbindung der Formel II'

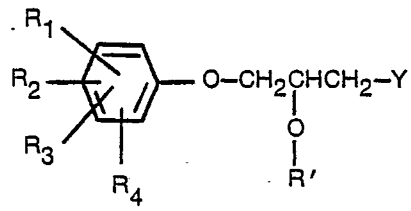

(II')

in welcher

$R_1$, $R_2$, $R_3$, $R_4$ die obengenannten Bedeutungen haben,

R' Wasserstoff oder eine Schutzgruppe,

Y eine reaktive Gruppe oder

R' und Y gemeinsam einen Valenzstrich bedeuten, mit einer Verbindung der Formel III'

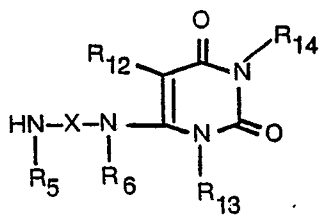

(III')

in welcher

$R_5$, X, $R_6$, $R_{12}$, $R_{13}$, $R_{14}$ die obengenannten Bedeutungen haben, umsetzt oder

b) eine Verbindung der Formel IV'

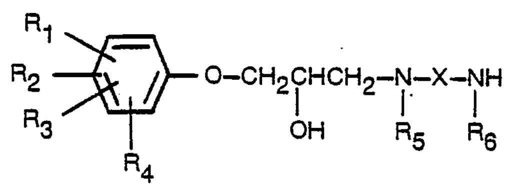

(IV')

in welcher

$R_1$, $R_2$, $R_3$, $R_4$, $R_5$, X, $R_6$ die obengenannten Bedeutungen haben, mit einer Verbindung der Formel V'

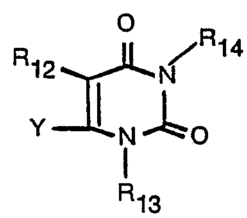

(V')

in welcher

$R_{12}$, $R_{13}$, $R_{14}$ die oben angegebenen Bedeutungen haben und

Y eine reaktive Gruppe darstellt, umsetzt oder

c) eine Verbindung der Formel VI'

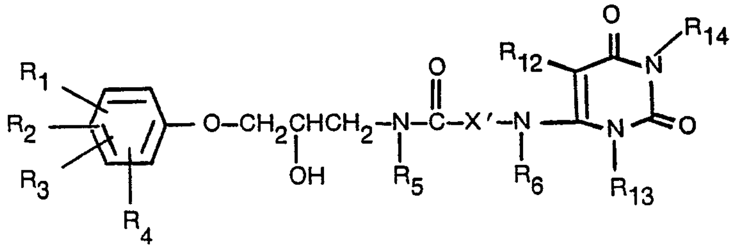

(VI')

in welcher

$R_1$, $R_2$, $R_3$, $R_4$, $R_5$, $R_6$, $R_{12}$, $R_{13}$, $R_{14}$ die oben angegebenen Bedeutung haben und

X' eine geradkettige Methylen- oder Ethylenkette bedeutet,

reduziert und gegebenenfalls vorhandene Schutzgruppen durch Hydrolyse oder Hydrogenolyse abspaltet,

und gegebenenfalls anschließend die erhaltenen Verbindungen der allgemeinen Formel I' in andere Verbindungen der allgemeinen Formel I' in an sich bekannter Weise überführt,

gegebenenfalls ein racemisches Gemisch von Verbindungen der allgemeinen Formel I' in an sich bekannter Weise in optisch aktive Formen trennt

und gegebenenfalls das resultierende Aryloxypropanolamin der allgemeinen Formel I' durch Umsetzung mit einer anorganischen oder organischen Säure in ein pharmakologisch unbedenkliches Salz überführt.

9. Verfahren zur Herstellung von Aryloxypropanolaminen der allgemeinen Formel I''

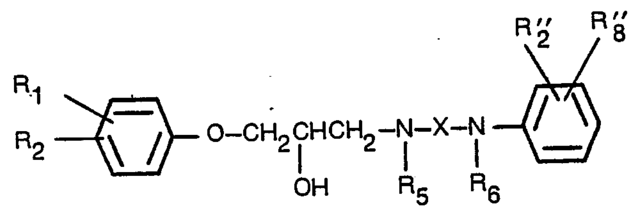

(I''),

in welcher

$R_1$ und $R_2$ gleich oder verschieden sind und ein Wasserstoffatom, $C_1$—$C_5$-Acyloxy, $C_1$—$C_4$-Alkoxy, Cyan, Carboxamido, Halogen, Hydroxy, Allyloxy, Phenyl-$C_1$—$C_4$-alkoxy, $C_1$—$C_4$-Alkyl,

$R_5$ und $R_6$ Wasserstoff,

X eine geradkettige Ethylen- oder Propylengruppe,

$R_7''$ und $R_8''$ gleich oder verschieden sind und Alkyl mit 1—4 C—Atomen bedeuten, mit der Maßgabe, daß $R_1$ und $R_2$ nicht gleichzeitig Wasserstoff sein dürfen, deren optisch aktive Formen, racemische Gemische sowie deren pharmakologisch verträgliche Salze, dadurch gekennzeichnet, daß man in an sich bekannter Weise entweder

a) eine Verbindung der Formel II''

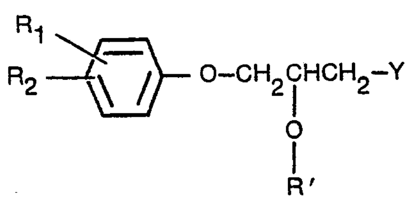

(II'')

in welcher

$R_1$, $R_2$ die obengenannten Bedeutungen haben,

R' Wasserstoff oder eine Schutzgruppe,

Y eine reaktive Gruppe oder

R' und Y gemeinsam einen Valenzstrich bedeuten, mit einer Verbindung der Formel III''

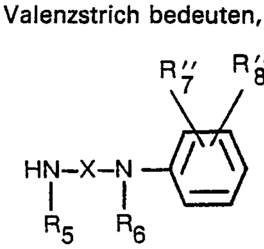

(III'')

in welcher

$R_5$, X, $R_6$, $R_7''$, $R_8''$ die obengenannten Bedeutungen haben, umsetzt oder

b) eine Verbindung der Formel IV''

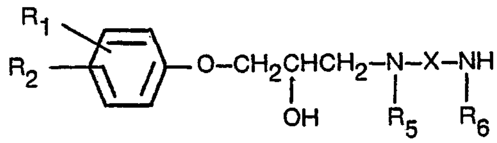

(IV'')

in welcher

$R_1$, $R_2$, $R_5$, X, $R_6$ die obengenannten Bedeutungen haben, mit einer Verbindung der Formel V''

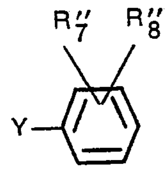

(V'')

in welcher

$R_7''$, $R_8''$ die oben angegebenen Bedeutungen haben und

Y eine reaktive Gruppe darstellt, umsetzt oder

c) eine Verbindung der Formel VI''

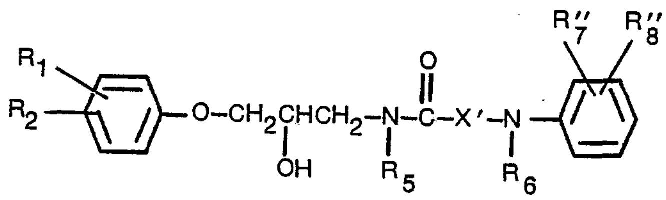

(VI'')

in welcher

$R_1$, $R_2$, $R_5$, $R_6$, $R_7''$, $R_8''$ die oben angegebenen Bedeutungen haben und

X' eine geradkettige Methylen- oder Ethylenkette bedeutet,

reduziert und gegebenenfalls vorhandene Schutzgruppen durch Hydrolyse oder Hydrogenolyse abspaltet,

und gegebenenfalls anschließend die erhalten Verbindungen der allgemeinen Formel I'' in andere Verbindungen

der allgemeinen Formel I'' in an sich bekannter Weise überführt,

gegebenenfalls ein racemisches Gemisch von Verbindungen der allgemeinen Formel I'' in an sich bekannter Weise in optisch aktive Formen trennt

und gegebenenfalls das resultierende Aryloxypropanolamin der allgemeinen Formel I'' durch Umsetzung mit einer anorganischen oder organischen Säure in ein pharmakologisch unbedenkliches Salz überführt.

10. Verfahren zur Herstellung von

1) 1-Phenoxy-3-[2-(1.3.6-trimethyl-pyrimidin-2.4-dion-5-yl-amino)-ethylamino]-propan-2-ol,

2) 1-Phenoxy-3-[2-(1.3.5-trimethyl-pyrimidin-4-on-2-thion-6-yl-amino)-ethylamino]-propan-2-ol sowie dessen Fumarat und

3) 1-Phenoxy-3-[2-(1.3.5-trimethyl-pyrimidin-2-on-4-thion-6-yl-amino)-ethylamino]-propan-2-ol sowie dessen Fumarat,

dadurch gekennzeichnet, daß man in an sich bekannter Weise entweder

a) eine Verbindung der Formel II'''

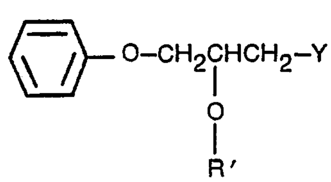

(II''')

in welcher

R' Wasserstoff oder eine Schutzgruppe,

Y eine reaktive Gruppe oder

R' und Y gemeinsam einen Valenzstrich bedeuten, mit einer Verbindung der Formel III'''

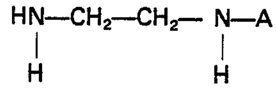

(III''')

in welcher

A einen 1.3.6-Trimethyl-pyrimidin-2.4-dion-5-yl-, einen 1.3.5-Trimethyl-pyrimidin-4-on-2-thion-6-yl-, bzw. einen 1.3.5-Trimethyl-pyrimidin-2-on-4-thion-6-yl-Rest darstellt, umsetzt oder

b) eine Verbindung der Formel IV'''

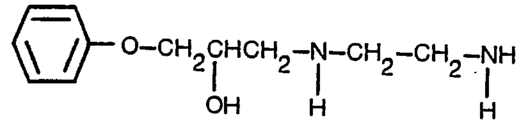

(IV''')

mit einer Verbindung der Formel V'''

$$Y—A \qquad (V''')$$

in welcher

A die oben angegebene Bedeutung hat und

Y eine reaktive Gruppe darstellt, umsetzt oder

c) eine Verbindung der Formel VI'''

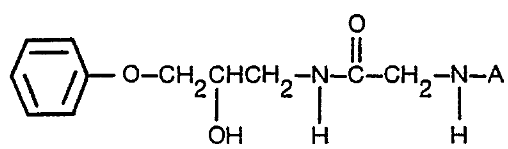

(VI''')

in welcher

A die oben angegebene Bedeutung hat,

reduziert und gegebenenfalls vorhandene Schutzgruppen durch Hydrolyse oder Hydrogenolyse abspaltet und anschließend gegebenenfalls die erhaltenen Verbindungen 2) oder 3) in das Fumarat überführt.

11. Aryloxypropanolamine gemäß einem der Anspruche 1 bis 6 oder deren Salze zur Verwendung bei der Bekämpfung von Herz- und Kreislauferkrankungen sowie zu deren Prophylaxe.

12. Arzneimittel enthaltend einen Wirkstoff gemäß einem der Ansprüche 1 bis 6 sowie an sich bekannte pharmakologisch verträgliche Trägerstoffe.

## Claims

1. Aryloxypropanolamines of the general formula I

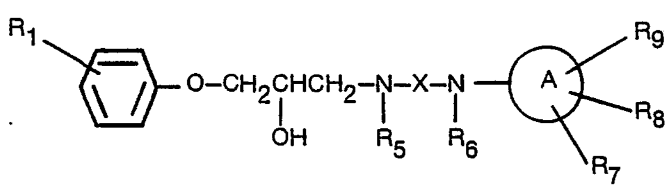

(I)

in which $R_1$ signifies a hydrogen atom, $C_1$—$C_4$-alkyl, cyano, carboxamido, halogen, hydroxyl, $C_1$—$C_5$-acyloxy, $C_1$—$C_4$-alkoxy, allyloxy, phenyl-$C_1$—$C_4$-alkoxy, $R_5$ and $R_6$ are the same or diferent and signify a hydrogen atom or $C_1$—$C_4$-alkyl, X signifies a straight-chained ethylene or propylene group, A signifies a residue connected with a ring carbon atom to the nitrogen selected from the group pyrazole, pyridine, indazole, quinoxaline, pyrazolone and purine, $R_7$, $R_8$ and $R_9$ are the same or different and signify hydrogen, $C_1$—$C_4$-alkyl or allyl, their optically-active forms, racemic mixtures, as well as their pharmacologically acceptable salts.

2. Aryloxypropanolamines of the general formula I'

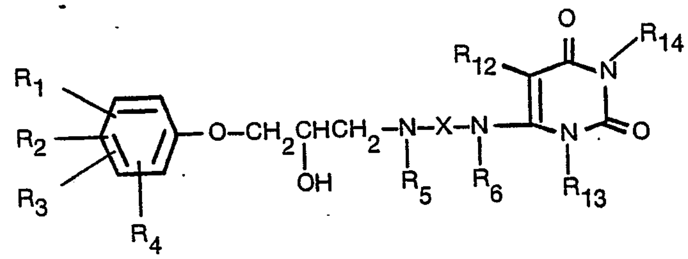

(I'),

in which $R_1$, $R_2$, $R_3$, $R_4$ are the same or different and signify a hydrogen atom, $C_1$—$C_4$-alkyl, cyano, carboxamido, halogen, hydroxyl, $C_1$—$C_5$-acyloxy, $C_1$—$C_4$-alkoxy, allyloxy, phenyl-$C_1$—$C_4$-alkoxy, $R_5$ and $R_6$ are the same or different and signify a hydrogen atom or $C_1$—$C_4$-alkyl, X signifies a straight-chained ethylene or propylene group, $R_{12}$ signifies cyano, $C_1$—$C_4$-alkyl or phenyl, $R_{13}$ signifies $C_1$—$C_4$-alkyl and $R_{14}$ signifies $C_1$—$C_4$-alkyl, their optically-active forms, racemic mixtures, as well as their pharmacologically acceptable salts.

3. 1-Phenoxy-3-[2-(1,3,6-trimethylpyrimidine-2,4-dione-5-ylamino)-ethylamino]-propan-2-ol.

4. 1-Phenoxy-3-[2-(1,3,5-trimethylpyrimidin-4-one-2-thione-6-ylamino)-ethylamino]-propan-2-ol, as well as its fumarate.

5. 1-Phenoxy-3-[2-(1,3,5-trimethylpyrimidin-2-one-4-thione-6-ylamino)-ethylamino]-propan-2-ol, as well as its fumarate.

6. Aryloxypropanolamines of the general formula I''

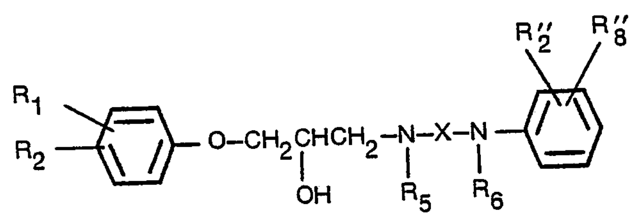

(I''),

in which $R_1$ and $R_2$ are the same or different and signify a hydrogen atom, $C_1$—$C_5$-acyloxy, $C_1$—$C_4$-alkoxy, cyano, carboxamido, halogen, hydroxyl, allyloxy, phenyl-$C_1$—$C_4$-alkoxy, $C_1$—$C_4$-alkyl, $R_5$ and $R_6$ signify hydrogen, X signifies a straight-chained ethylene or propylene group, $R''_7$ and $R''_8$ are the same or different and signify alkyl with 1—4 C-atoms, with the proviso that $R_1$ and $R_2$ may not simultaneously be hydrogen, their optically-active forms, racemic mixtures, as well as their pharmacologically acceptable salts.

7. Process for the preparation of aryloxypropanolamines of the general formula I

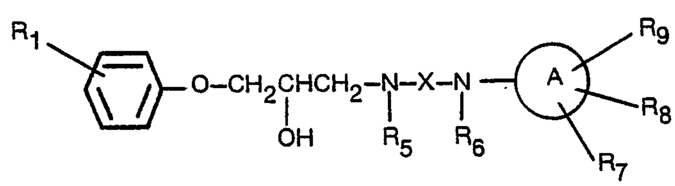

(I)

in which $R_1$ signifies a hydrogen atom, $C_1$—$C_4$-alkyl, cyano, carboxamido, halogen, hydroxyl, $C_1$—$C_5$-acyloxy, $C_1$—$C_4$-alkoxy, allyloxy, phenyl-$C_1$—$C_4$-alkoxy, $R_5$ and $R_6$ are the same or different and signify a hydrogen atom or $C_1$—$C_4$-alkyl, X signifies a straight-chained ethylene or propylene group, A signifies a residue connected with a ring carbon atom to the nitrogen, selected from the group pyrazole, pyridine, indazole, quinoxaline, pyrazolone and purine, $R_7$, $R_8$ and $R_9$ are the same or different and signify hydrogen, $C_1$—$C_4$-alkyl or allyl, their optically-active forms, racemic mixtures, as well as their pharmacologically acceptable salts, characterised in that, in per se known manner, one either

a) reacts a compound of the formula II

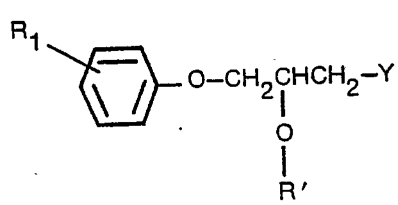

(II)

in which $R_1$ has the above-mentioned meaning, R′ signifies hydrogen or a protective group, Y a reactive group or R′ and Y together signify a valency bond, with a compound of the general formula III

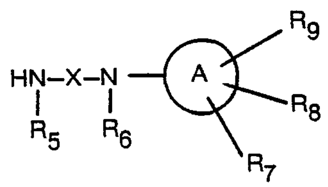

(III)

in which $R_5$, X, $R_6$, A, $R_7$, $R_8$, $R_9$ have the above-mentioned meanings, or

b) reacts a compound of the formula IV

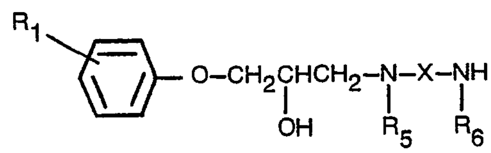

(IV)

in which $R_1$, $R_5$, X, $R_6$ have the above-mentioned meanings, with a compound of the formula V

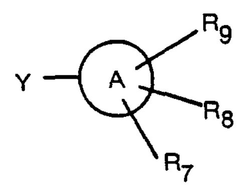

(V)

in which A, $R_7$, $R_8$, $R_9$ have the above-given meanings and Y represents a reactive group, or

c) reduces a compound of the formula VI

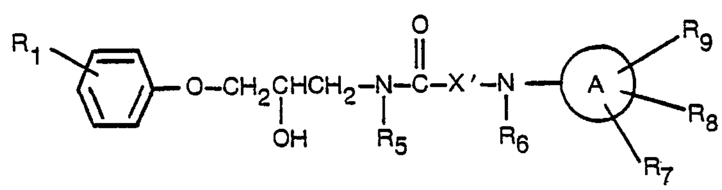

(VI)

in which A, $R_1$, $R_5$, $R_6$, $R_7$, $R_8$, $R_9$ have the above-given meanings and X′ signifies a straight-chained methylene or ethylene group, and splits off possibly present protective groups by hydrolysis or hydrogenolysis, and possible subsequently converts the compounds obtained of the general formula I into other compounds of the general formula I in per se known manner, optionally separates a racemic mixture of compounds of the general formula I in per se known manner into optically-active forms and possibly converts the resulting aryloxypropanolamine of the general formula I, by reaction with an inorganic or organic acid, into a pharmacologically acceptable salt.

8. Process for the preparation of aryloxypropanolamines of the general formula I′

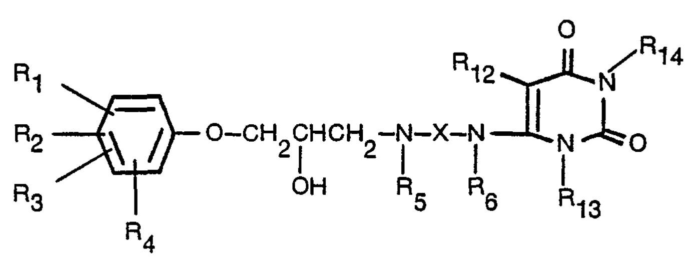

(I′),

in which $R_1$, $R_2$, $R_3$, $R_4$ are the same or different and signify a hydrogen atom, $C_1$—$C_4$-alkyl, cyano, carboxamido, halogen, hydroxyl, $C_1$—$C_5$-acyloxy, $C_1$—$C_4$-alkoxy, allyloxy, phenyl-$C_1$—$C_4$-alkoxy, $R_5$ and $R_6$ are the same or different and signify a hydrogen atom or $C_1$—$C_4$ alkyl, X signifies a straight-chained ethylene or

propylene group, $R_{12}$ signifies cyano, $C_1$—$C_4$-alkyl or phenyl, $R_{13}$ signifies $C_1$—$C_4$-alkyl and $R_{14}$ signifies $C_1$—$C_4$-alkyl, their optically-active forms, racemic mixtures, as well as their pharmacologically acceptable salts, characterised in that, in per se known manner, one either

a) reacts a compound of the formula II'

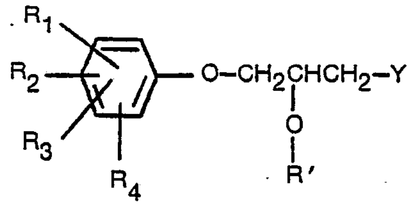

(II')

in which $R_1$, $R_2$, $R_3$, $R_4$ have the above-mentioned meanings, R' signifies hydrogen or a protective group, Y signifies a reactive group or R' and Y together signify a valency bond, with a compound of the formula III'

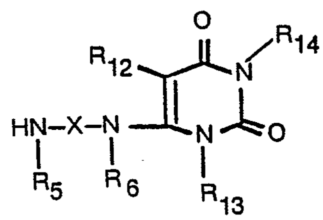

(III')

in which $R_5$, X, $R_6$, $R_{12}$, $R_{13}$, $R_{14}$ have the above-given meanings, or

b) reacts a compound of the formula IV'

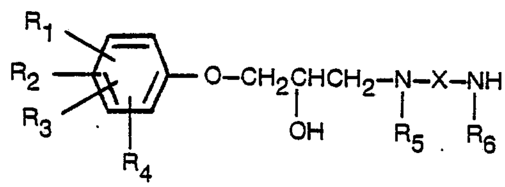

(IV')

in which $R_1$, $R_2$, $R_3$, $R_4$, $R_5$, X, $R_6$ have the above-mentioned meanings, with a compound of the formula V'

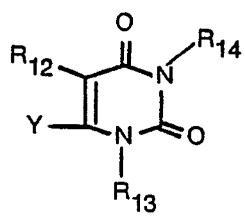

(V')

in which $R_{12}$, $R_{13}$, $R_{14}$ have the above-mentioned meanings and Y represents a reactive group, or

c) reduces a compound of the formula VI'

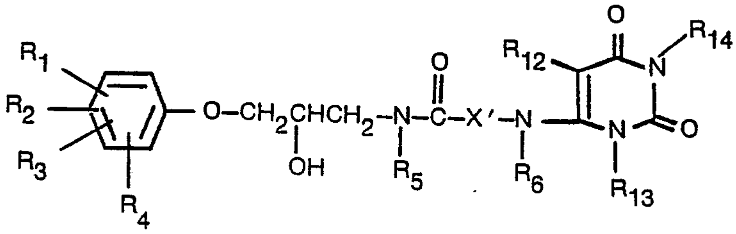

(VI')

in which $R_1$, $R_2$, $R_3$, $R_4$, $R_5$, $R_6$, $R_{12}$, $R_{13}$, $R_{14}$ have the above-given meanings and X' signifies a straight-chained methylene or ethylene chain, and splits off possibly present protective groups by hydrolysis or hydrogenolysis, and possibly subsequently converts the compounds obtained of the general formula I' into other compounds of the general formula I' in per se known manner, optionally separates a racemic mixture of compounds of the general formula I' in per se known manner into optically-active forms and optionally converts the resulting aryloxypropanolamine of the general formula I' by reaction with an inorganic or organic acid into a pharmacologically acceptable salt.

9. Process for the preparation of aryloxypropanolamines of the general formula I''

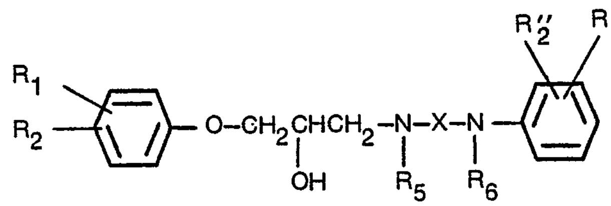

(I''),

in which $R_1$ and $R_2$ are the same or different and signify a hydrogen atom, $C_1$—$C_5$-acyloxy, $C_1$—$C_4$-alkoxy, cyano, carboxamido, halogen, hydroxyl, allyloxy, phenyl-$C_1$—$C_4$-alkoxy, $C_1$—$C_4$-alkyl, $R_5$ and $R_6$ signify hydrogen, X signifies a straight-chained ethylene or propylene group, $R''_7$ and $R''_8$ are the same or different and signify alkyl with 1—4 C-atoms, with the proviso that $R_1$ and $R_2$ may not simultaneously be hydrogen, their optically-active forms, racemic mixtures, as well as their pharmacologically acceptable salts, characterised in that, in per se known manner, one either

a) reacts a compound of the formula II''

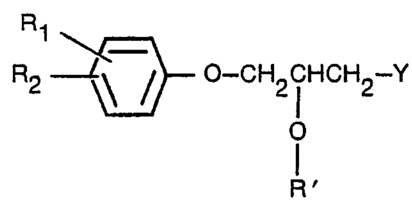

(II'')

in which $R_1$, $R_2$ have the above-mentioned meanings, R' signifies hydrogen or a protective group, Y signifies a reactive group or R' and Y together signify a valency bond, with a compound of the formula III''

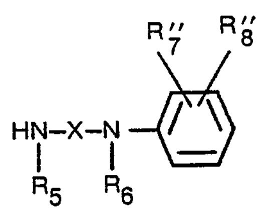

(III'')

in which $R_5$, X, $R_6$, $R''_7$, $R''_8$ have the above-mentioned meanings, or

b) reacts a compound of the formula IV''

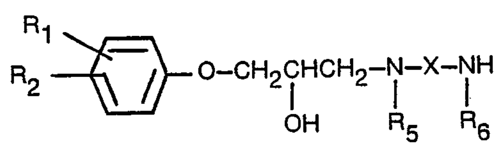

(IV'')

in which $R_1$, $R_2$, $R_5$, X, $R_6$ have the above-mentioned meanings, with a compound of the formula V''

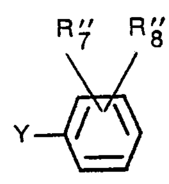

(V'')

in which $R''_7$, $R''_8$ have the above-given meanings and Y represents a reactive group, or

c) reduces a compound of the formula VI''

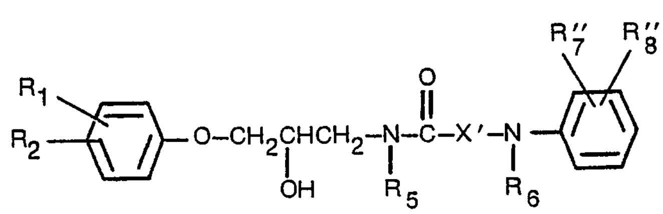

(VI'')

in which $R_1$, $R_2$, $R_5$, $R_6$, $R''_7$, $R''_8$ have the above-given meanings and X' signifies a straight-chained methylene or ethylene chain, and splits off possibly present protective groups by hydrolysis or hydrogenolysis, and possibly subsequently converts the compounds obtained of the general formula I'' into other compounds of the general formula I'' in per se known manner, optionally separates a racemic mixture of compounds of the general formula I'' in per se known manner into optionally-active forms and optionally converts the resulting aryloxypropanolamine of the general formula I'' by reaction with an inorganic or organic acid into a pharmacologically acceptable salt.

10. Process for the preparation of

1) 1-phenoxy-3-[2-(1,3,6-trimethylpyrimidine-2,4-dione-5-ylamino)-ethylamino]-propan-2-ol,

2) 1-phenoxy-3-[2-(1,3,5-trimethylpyrimidin-4-one-2-thione-6-ylamino)-ethylamino]-propan-2-ol, as well as its fumarate and

3) 1-phenoxy-3-[2-(1,3,5-trimethylpyrimidin-2-one-4-thione-6-ylamino)-ethylamino]-propan-2-ol, as well as its fumarate,

characterised in that in per se known manner one either

a) reacts a compound of the formula II'''

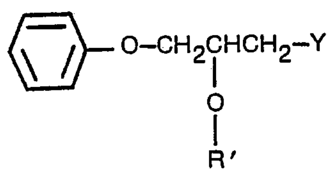

(II''')

in which R' signifies hydrogen or a protective group, Y signifies a reactive group or R' and Y together signify a valency bond, with a compound of the formula III'''

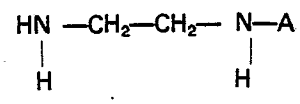

(III''')

in which A represents a 1,3,6-trimethylpyrimidine-2,4-dione-5-yl, a 1,3,5-trimethylpyrimidin-4-one-2-thione-6-yl or a 1,3,5-trimethylpyrimidin-2-one-4-thione-6-yl radical, or

b) reacts a compound of the formula IV'''

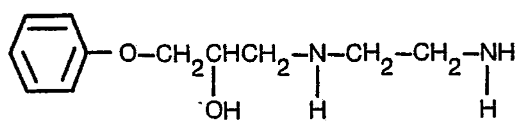

(IV''')

with a compound of the formula V'''

Y—A (V''')

in which A has the above-given meaning and Y represents a reactive group, or

c) reduces a compound of the formula VI'''

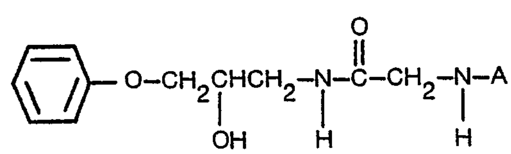

(VI''')

in which A has the above-given meaning, and splits off possibly present protective groups by hydrolysis or hydrogenolysis and subsequently optionally converts the compounds 2) or 3) obtained into the fumarate.

11. Aryloxypropanolamines according to one of claims 1 to 6 or their salts for use in the combating of heart and circulatory diseases, as well as the prophylaxis thereof.

12. Medicaments containing an active material according to one of claims 1 to 6, as well as per se known pharmacologically acceptable carrier materials.

**Revendications**

1. Aryloxypropanolamines de formule générale I

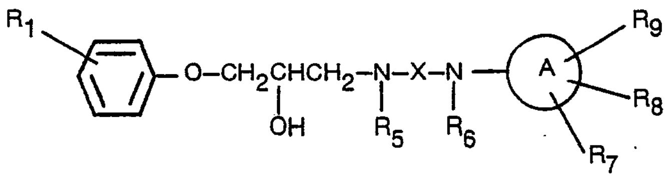

(I)

dans laquelle:

$R_1$ est un atome d'hydrogène, de l'alkyle $C_1$—$C_4$, du cyano, du carboxamido, de l'halogène, de l'hydroxyle, de l'acyloxy $C_1$—$C_5$, de l'alcoxy $C_1$—$C_4$, de l'allyloxy, du phényl-alcoxy $C_1$—$C_4$,

$R_5$ et $R_6$ sont identiques ou différents et représentent un atome d'hydrogène ou de l'alkyle $C_1$—$C_4$

X est un groupe éthylène ou propylène à chaîne droite,

A est un reste dont un atome de carbone du noyau est lié à l'azote, choisi dans le groupe comprenant le pyrazole, la pyridine, l'indazole, la quinoxaline, la pyrazolone et la purine,

$R_7$, $R_8$ et $R_9$ sont identiques ou différents et représentent de l'hydrogène de l'alkyle $C_1$—$C_4$ ou de l'allyle, leurs formes optiquement actives, leurs mélanges racémiquesainsi que leurs sels pharmacologiquement compatibles.

2. Aryloxypropanolamines de formule générale I'

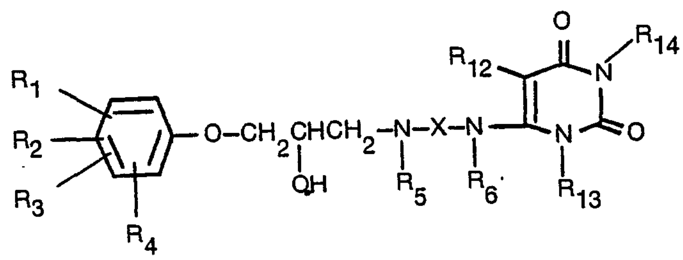

(I'),

dans laquelle

$R_1$, $R_2$, $R_3$, $R_4$ sont identiques ou différents et représentent un atome d'hydrogène, de l'alkyle $C_1$—$C_4$, du cyano, du carboxamido, de l'halogène, de l'hydroxyle, de l'acyloxy $C_1$—$C_5$, de l'alcoxy $C_1$—$C_4$, de l'allyloxy, du phényl-alcoxy-$C_1$—$C_4$

$R_5$ et $R_6$ sont identiques ou différents et représentent un atome d'hydrogène ou de l'alkyle $C_1$—$C_4$,

X est un groupe éthylène ou propylène à chaîne droite

$R_{12}$ est du cyano, de l'alkyle $C_1$—$C_4$ ou du phényle

$R_{13}$ de l'alkyle $C_1$—$C_4$ et

$R_{14}$ de l'alkyle $C_1$—$C_4$,

leurs formes optiquement actives, leurs mélanges racémiques ainsi que leurs sels pharmacologiquement compatibles.

3. Le 1-phénoxy 3-[2-(1,3,6-triméthyl-pyrimidine 2,4-dione-5-yl-amino)éthylamino]propane-2-ol.

4. Le 1-phénoxy 3-[2-(1,3,5-triméthyl-pyrimidine-4-one-2-thione-6-yl-amino)éthylamino]propane-2-ol, ainsi que son fumarate

5. Le 1-phénoxy 3-[2-(1,3,5-triméthyl-pyrimidine-2-one-4-thione-6-yl-amino)éthylamino]propane-2-ol, ainsi que son fumarate.

6. Aryloxypropanolamines de formule générale I''

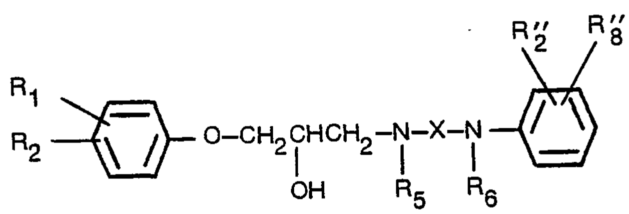

(I''),

dans laquelle

$R_1$ et $R_2$ sont identiques ou différents et représentent un atome d'hydrogène, de l'acyloxy $C_1$—$C_5$, de l'alcoxy $C_1$—$C_4$, du cyano, du carboxamido, de l'halogène, de l'hydoxyle, de l'allyloxy, du phényl-alcoxy $C_1$—$C_4$, de l'alkyle $C_1$—$C_4$,

$R_5$ et $R_6$ sont de l'hydrogène

X est un groupe éthylène ou propylène à chaîne droite,

$R_7''$ et $R_8''$ sont identiques ou différents et représentent de l'alkyle ayant de 1 à 4 atomes de carbone, sous la condition que $R_1$ et $R_2$ ne doivent pas représenter simultanément de l'hydrogène, leurs formes optiquement actives, leurs mélanges racémiques ainsi que leurs sels pharmacologiquement compatibles.

7. Procédé de préparation d'aryloxypropanolamines de formule générale I

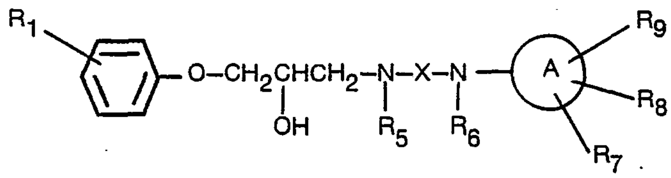

(I)

dans laquelle

$R_1$ est un atome d'hydrogène, de l'alkyle $C_1$—$C_4$, du cyano, du carboxamido, de l'halogène, de l'hydroxyle, de l'acyloxy $C_1$—$C_5$, de l'alcoxy $C_1$—$C_4$, de l'allyloxy, du phényl-alcoxy $C_1$—$C_4$,

$R_5$ et $R_6$ sont identiques ou différents et représentent un atome d'hydrogène ou de l'alkyle $C_1$—$C_4$,

X est un groupe éthylène ou propylène à chaîne droite,

A est un reste dont un atome de carbone du noyau est lié à l'azote, choisi dans le groupe comprenant le pyrazole, la pyridine, l'indazole, la quinoxaline, la pyrazolone et la purine

$R_7$, $R_8$ et $R_9$ sont identiques ou différents et représentent de l'hydrogène de l'alkyle $C_1$—$C_4$ ou de l'allyle, leurs formes optiquement actives, leurs mélanges racémiques ainsi que leurs sels pharmacologiquement compatibles, caractérisé en ce que de façon en soit connue:

a) on fait réagir un composé de formule II

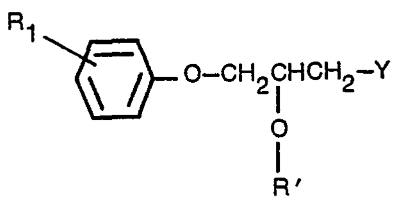

(II)

dans laquelle

$R_1$ a la signification ci-dessus donnée

R' est de l'hydrogène ou un groupe de protection

Y est un groupe réactif, ou

R' et Y désignent ensemble un trait de valence avec un composé de formule III

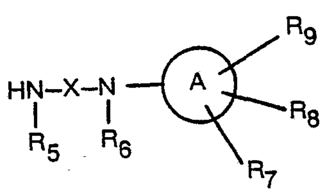

(III)

dans laquelle

$R_5$, X, $R_6$, A, $R_7$, $R_8$, $R_9$ ont les significations ci-dessus données, ou,

b) on fait réagir un composé de formule IV

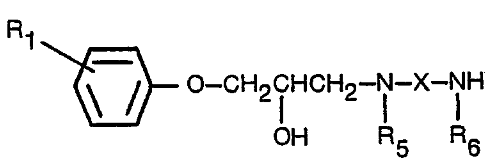

(IV)

dans laquelle

$R_1$, $R_5$, X, $R_6$ ont les significations ci-dessus données, avec un composé de formule V

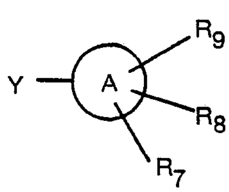

(V)

dans laquelle

A, $R_7$, $R_8$, $R_9$ ont les significations données ci-dessus et
Y est un groupe réactif, ou
c) on soumet un composé de formule VI

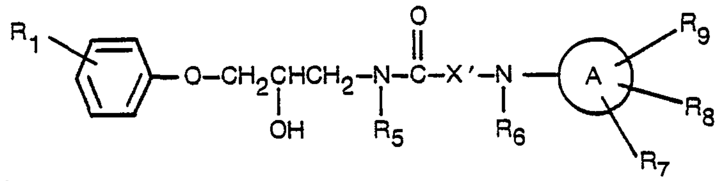

(VI)

dans laquelle:

A, $R_1$, $R_5$, $R_6$, $R_7$, $R_8$, $R_9$ ont les significations données ci-dessus, et X' est un groupe méthylène ou éthylène à chaîne droite à une réduction et on élimine les groupes de protection éventuellement présents par hydrolyse ou hydrogénolyse, et on transforme éventuellement ensuite, de façon en soie connue, les composés de formule générale I obtenus en d'autres composés de formule générale I, on sépare éventuellement, de façon en soi connue, en mélange racémique de composés de formule générale I en formes optiquement actives, et on transforme éventuellement l'aryloxypropanolamine de formule générale I, ainsi obtenue par réaction avec un acid minéral ou organique en un sel pharmacologiquement acceptable.

8. Procédé de préparation d'aryloxypropanolamines de formule générale I'

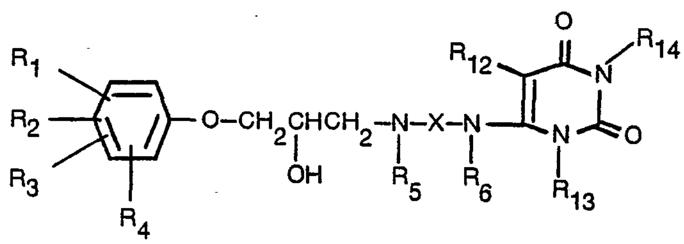

(I'),

dans laquelle:

$R_1$, $R_2$, $R_3$, $R_4$ sont identiques ou différents et représentent un atome d'hydrogène, de l'alkyle $C_1$—$C_4$, du cyano, du carboxamido, de l'halogène, de l'hydroxyle, de l'acyloxy $C_1$—$C_5$, de l'alcoxy $C_1$—$C_4$, de l'allyloxy, du phényl-alcoxy-$C_1$—$C_4$

$R_5$ et $R_6$ sont identiques ou différents et représentent un atome d'hydrogène ou de l'alkyle $C_1$—$C_4$,
X est un groupe éthylène ou propylène à chaîne droite
$R_{12}$ est du cyano, de l'alkyle $C_1$—$C_4$ ou du phényle
$R_{13}$ de l'alkyle $C_1$—$C_4$, et
$R_{14}$ de l'alkyle $C_1$—$C_4$,

leurs formes optiquement actives, leurs mélanges racémiques ainsi que leurs sels pharmacologiquement compatibles, caractérisé en ce que de façon ce soit connue,

a) on fait réagir un composé de formule II'

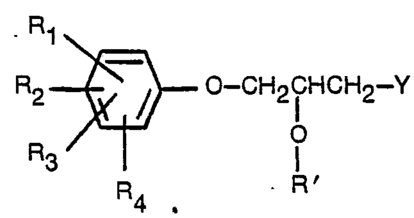

(II')

dans laquelle:

$R_1$, $R_2$, $R_3$, $R_4$ ont les signfications données ci-dessus,
R' est de l'hydrogène ou un groupe de protection,
Y un groupe réactif ou
R' et Y représentent ensemble un trait de valence avec un composé de formule III';

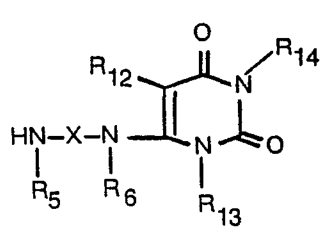

(III')

dans laquelle

$R_5$, X, $R_6$, $R_{12}$, $R_{13}$, $R_{14}$ ont les significations ci-dessus données, ou,

b) on fait réagir un composé de formule IV'

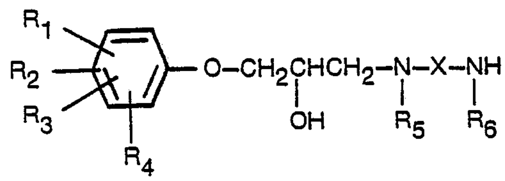

(IV')

dans laquelle

$R_1$, $R_2$, $R_3$, $R_4$, $R_5$, X, $R_6$ ont les significations ci-dessus données avec un composé de formule V'

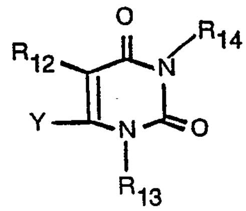

(V')

dans laquelle

$R_{12}$, $R_{13}$, $R_{14}$ ont les significations ci-dessus données et, Y est un groupe réactif, ou,

c) on soumet un composé de formule VI'

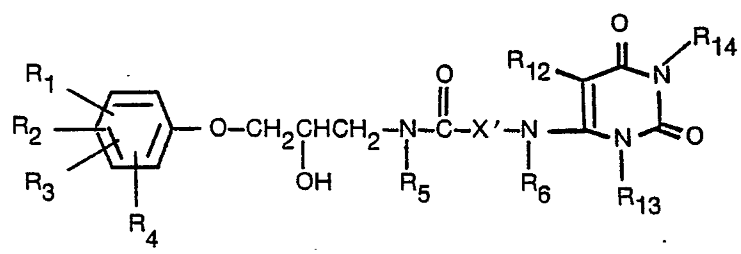

(VI')

dans laquelle

$R_1$, $R_2$, $R_3$, $R_4$, $R_5$, $R_6$, $R_{12}$, $R_{13}$, $R_{14}$ ont la signification ci-dessus donnée et X' est une chaîne méthylène ou éthylène droite, à une réduction et on élimine éventuellement les groupes de protection présents par hydrolyse ou hydrogénolyse, et on transforme éventuellement ensuite, de façon connue, les composés de formule générale I' obtenus en d'autres composés de formule générale I' on sépare éventuellement de façon en soi connue un mélange racémique de composés de formule générale I' en formes optiquement actives, et on transforme éventuellement, de façon en soi connue, l'aryloxypropanol-amine de formule générale I'ainsi obtenue par réaction avec un acide minéral ou organique en un sel pharmacologiquement acceptable.

9. Procédé de préparation d'aryloxypropanolamines de formule générale I''

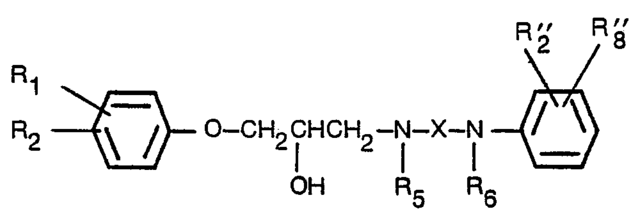

(I''),

dans laquelle

$R_1$ et $R_2$ sont identiques ou différents et représentent un atome d'hydrogène, de l'acyloxy $C_1$—$C_5$, de l'alcoxy $C_1$—$C_4$, du cyano, du carboxamido, de l'halogène, de l'hydroxyle, de l'allyloxy, du phényl-alcoxy $C_1$—$C_4$, de l'alkyle $C_1$—$C_4$,

$R_5$ et $R_6$ sont de l'hydrogène

X est un groupe éthylène ou propylène à chaîne droite,

$R_7''$ et $R_8''$ sont identiques ou différents et représentent de l'alkyle ayant de 1 à 4 atomes de carbone, sous la condition que $R_1$ et $R_2$ ne doivent pas représenter simultanément de l'hydrogène, leurs formes optiquement actives, leurs mélanges racémiques ainsi que leurs sels pharmacologiquement compatibles, caractérisé en ce que, de façon en soi connue,

a) on fait réagir un composé de formule II''

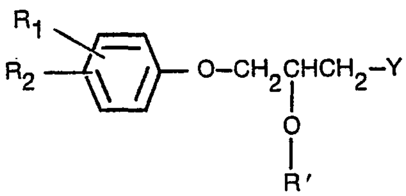

(II'')

dans laquelle

$R_1$, $R_2$ ont les significations données ci-dessus

R' est de l'hydrogène ou un groupe de protection,

Y est un groupe réactif ou,

R' et Y désignent ensemble un trait de valence,

avec un composé de formule III''

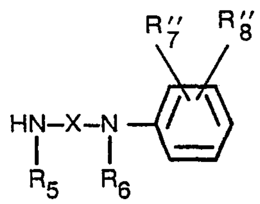

(III'')

dans laquelle

$R_5$, X, $R_6$, $R_7''$, $R_8''$ ont les significations données ci-dessus, ou

b) on fait réagir un composé de formule IV''

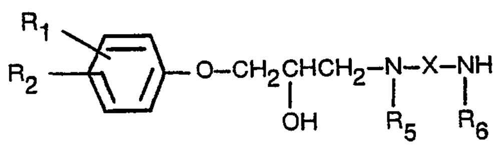

(IV'')

dans laquelle

$R_1$, $R_2$, $R_5$, X, $R_6$ ont les significations données ci-dessus, avec un composé de formule V'':

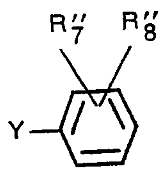

(V'')

dans laquelle

$R_7''$, $R_8''$ ont les significations ci-dessus indiquées et,

Y est un groupe réactif, ou,

c) ou soumet un composé de formule VI''

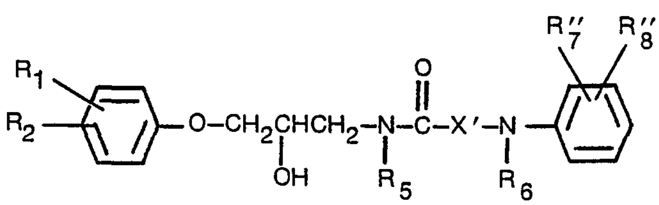

(VI'')

dans laquelle

$R_1$, $R_2$, $R_5$, $R_6$, $R_7''$, $R_8''$ ont les significations indiquées ci-dessus et,

X' est un chaîne méthylène ou éthylène droite,

à une réduction et on élimine les groupes de protection éventuellement présents par hydrolyse ou hydrogénolyse,

et éventuellement on transforme ensuite, de façon en soi connue, les composés de formule générale I'' obtenus en d'autres composés de formule générale I'', on sépare éventuellement de façon en soi connue,

un mélange racémique de composés de formule générale I'' en formes optiquement actives, et on transforme éventuellement l'aryloxypropanolamine de formule générale I'' ainsi obtenue par réaction avec un acide minéral ou organique en un sel pharmacologiquement acceptable.

10. Procédé de préparation du:

1) 1-phénoxy 3-[2-(1,3,6-triméthyl-pyrimidine-2,4-dione-5-yl-amino)éthylamino]propane-2-ol,

2) 1-phénoxy 3-[2-(1,3,5-triméthyl-pyrimidine-4-one-2-thione-6-yl-amino)éthylamino]propane-2-ol ainsi que de son fumarate, et du

3) 1-phénoxy 3-[2-(1,3,5-triméthyl-pyrimidine-2-one-4-thione-6-yl-amino)éthylamino]propane-2-ol ainsi que de son fumarate,

caractérisé en ce que, de façon en soi connue,

a) on fait réagir un composé de formule II'''

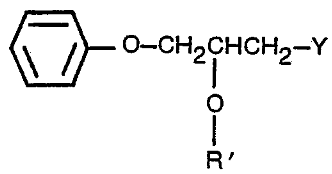

(II''')

dans laquelle:

'R' est de l'hydrogène ou un groupe de protection,

Y est un groupe réactif, ou

R' et Y' représentent ensemble un trait de valence,

avec un composé de formule III'''

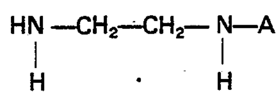

(III''')

dans laquelle

A est un reste 1,3,6-triméthyl-pyrimidine-2,4-dione-5-yle, un reste 1,3,5-triméthyl-pyrimidine-4-one-2-thione-6-yle ou un reste 1,3,5-triméthyl-pyrimidine-2-one-4-thione-6-yle, ou,

b) on fait réagir un composé de formule IV''':

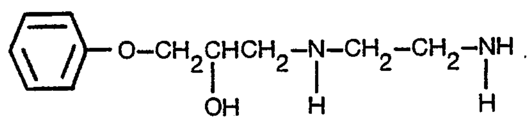

(IV''')

avec un composé de formule V'''

Y—A (V''')

dans laquelle

A a la signification donnée ci-dessus et

Y désigne une groupe réactif, ou,

c) ou soumet un composé de formule VI'''

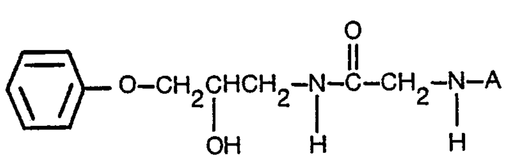

(VI''')

dans laquelle:

A a la signification ci-dessus indiquée à une réduction et on élimine des groupes de protection éventuellement présents par hydrolyse ou hydrogénolyse et on transforme ensuite éventuellement le composé 2) ou 3) obtenu en son fumarate.

11. Les aryloxypropanolamines selon l'une des revendications 1 à 6 ou leurs sels pour l'utilisation dans le traitement des maladies du coeur et de la circulation ainsi qu'à des fins prophylactiques.

12. Médicaments renfermant une substance active selon l'une des revendications 1 à 6 ainsi que des agents de support pharmacologiquement compatibles connus.